# EUROPÄISCHE PATENTANMELDUNG

(11) **EP 1 518 916 A1**
(43) Veröffentlichungstag der Anmeldung: **30.03.2005**
(21) Anmeldenummer: 03021803.6
(22) Anmeldetag: 26.09.2003
(51) Int. Cl.: C09K 15/20, C07B 63/04, C07C 7/00

(54) **Radikalfänger als stabilisatoren polymerisationsfähiger Verbindungen**

(71) Anmelder: BASF AKTIENGESELLSCHAFT, 67056 Ludwigshafen (DE)
(72) Erfinder: Dr. Frank Höfer, 67071 Ludwigshafen (DE); Dr. Sylke Haremza, 69151 Neckargemünd (DE); Dr. Gerhard Wagenblast, 67157 Wachenheim (DE); Dr. Volker Schliephake, 67105 Schifferstadt (DE); Dr. Ulrich Jäger, 67354 Römerberg (DE)

(57) **Zusammenfassung**

Verfahren zur Stabilisierung polymerisationsfähiger Verbindungen gegen Polymerisation bei der Aufarbeitung, Lagerung und/oder Transport, in dem man wenigstens einen Radikalfänger, der mindestens zwei Glycineinheiten enthält, verwendet, mit der Maßgabe, dass der Radikalfänger nicht ausgewählt ist aus Ethylendiamintetraessigsäure (EDTA), Diethylentriaminpentaessigsäure (DTPA), trans-1,2-Cyclohexandiamintetraessigsäure (CYDTA) und deren Alkali- und Erdalkalisalze.

## Beschreibung

Die vorliegende Erfindung beschreibt die Verwendung von Radikalfängern zur Stabilisierung polymerisationsfähiger Verbindungen gegen Polymerisation.

Es ist bekannt, dass polymerisationsfähige Verbindungen etwa durch Wärme oder Einwirkung von Licht oder Peroxiden leicht zur Polymerisation gebracht werden können. Da jedoch bei der Herstellung, Aufarbeitung, Lagerung und/oder Transport die Polymerisation aus sicherheitstechnischen und wirtschaftlichen Gründen vermindert oder verringert werden muss, besteht ein ständiger Bedarf an neuen, wirksamen Polymerisationsinhibitoren.

Insbesondere problematisch ist die chemische und/oder physikalische Bearbeitung bei der Aufarbeitung, wie beispielsweise durch Destillation oder Rektifikation, sowie anschließend die Lagerung und der Transport.

Bekannt sind eine Vielzahl von Stabilisatoren für polymerisationsfähige Verbindungen, insbesondere für Acrylsäure und Methacrylsäure, im folgenden (Meth)acrylsäure genannt, sowie deren Ester, im folgenden (Meth)acrylsäureester genannt.

In GB-A 1 601 979 wird die Stabilisierung einer wässrigen Lösung eines (Meth)acrylat-Salzes mit einem Nitrosophenolat in Gegenwart von EDTA als Chelator beschrieben.

In US 4,929,660 wird eine Klebstoffzusammensetzung offenbart, die ein radikalisches Acrylmonomer sowie einen Polymerisationsinhibitor, einen Metalchelator und einen Radikalfänger enthält. Bei diesem Radikalfänger handelt es sich ein N,N-Dialkyl- oder N,N-Diarylalkylhydroxylamin.

Die Stabilisierung von ungesättigten quaternären Ammoniumsalzen in Gegenwart von Metallfängern wird in US 5,912,383 beschrieben. Diese Metallfänger können Diethylentriaminpentaessigsäure und N-(Hydroxyethyl)ethylendiamintriessigsäure sowie die dazugehörigen Natriumsalze sein.

In der deutschen Offenlegungsschrift DE-A 199 20 796 wird ein Verfahren zur Herstellung von Isobornyl(meth)acrylat durch Umsetzung von Camphen mit (Meth)acrylsäure beschrieben, das ebenfalls in Gegenwart eines Chelatbildners durchgeführt wird. Als Chelatbildner offenbart diese Schrift Nitrilotriessigsäure, Ethylendiamintetraessigsäure, N-(2-Hydroxyethyl)ethylendiamintriessigsäure, 1,2-Cyclohexylendinitrilotetraessigsäure, Diethylentriaminpentaessigsäure, 3,6-Dioxaoctamethylendinitrilotetraessigsäure sowie die Alkalimetallsalze dieser Säuren.

WO 02/26685 beschreibt die Stabilisierung von Acrylmonomeren während der Destillation mit einem Stabilisator in Gegenwart von Sauerstoff mit einem Metallfänger, der ausgewählt ist aus Ethylendiamintetraessigsäure (EDTA), Diethylentriaminpentassigsäure (DTPA) und deren Na-Salz (Na₅DTPA) sowie trans-1,2-Cyclohexandiaminpentaessigsäure. Diese Metallfänger werden zur Komplexierung von freiem Eisen eingesetzt.

Die japanischen Offenlegungsschriften JP 05-295011 und JP 05-320205 beschreiben ebenfalls die von Acrylsäure in Gegenwart von EDTA, DTPA, CYDTA und deren Alkalisalze.

Aufgabe der vorliegenden Erfindung war daher, ein alternatives Verfahren zur Stabilisierung polymerisationsfähiger Verbindungen gegen Polymerisation bei der Aufarbeitung, Lagerung und/oder Transport zur Verfügung zu stellen.

Die Aufgabe wurde gelöst durch ein Verfahren zur Stabilisierung polymerisationsfähiger Verbindungen gegen Polymerisation bei der Aufarbeitung, Lagerung und/oder Transport, worin mindestens ein Radikalfänger, der mindestens zwei Glycineinheiten - enthält, verwendet wird, mit der Maßgabe, dass der Radikalfänger nicht ausgewählt ist aus Ethylendiamintetraessigsäure (EDTA), Diethylentriaminpentaessigsäure (DTPA), trans-1,2-Cyclohexandiamindiamintetraessigsäure (CYDTA) und deren Alkali- und Erdalkalisalze.

In einer bevorzugten Ausführungsform verwendet man wenigstens einen Radikalfänger, der mindestens zwei Glycineinheiten enthält, mit der Maßgabe, dass der Radikalfänger nicht ≥ 2 der folgenden Struktureinheiten aufweist: 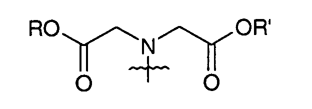 worin R, R' unabhängig voneinander Wasserstoff oder Metall sein können. Beispielsweise handelt es sich bei diesen Metallen um Alkalimetalle wie Natrium oder Kalium.

In einer besonders bevorzugten Ausführungsform verwendet man wenigstens Radikalfänger, der mindestens zwei Gylcineinheiten und mindestens eine Amid- und/oder Estereinheit enthält. Bevorzugt weist der Radikalfänger zwei Amideinheiten auf.

Ganz besonders bevorzugt werden solche Radikalfänger der allgemeinen Formel (I) verwendet:

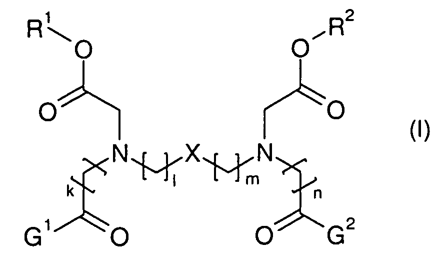

Im einzelnen können sein:
G¹ kann NR³R⁴ oder OR⁷ und G² kann NR⁵R⁶ oder OR⁸ sein.
X kann C₁-C₂₀-Alkyl, NCH₂COOR⁹, NR¹⁰, O, S, PR¹¹, Se, SiOR¹²R¹³ oder Aryl sein, wobei die genannten Substituenten an beliebiger Position, allerdings nicht mehr als fünfmal, bevorzugt nicht mehr als viermal, besonders bevorzugt nicht mehr als dreimal durch ein oder mehrere Heteroatome und/oder Halogenatome substituiert sein können. Bevorzugt ist X eine C₁-C₂₀-Alkylgruppe oder NCHCOOR⁹, besonders bevorzugt ist X eine C₁-C₁₀-Alkylgruppe oder NCHCOOR⁹.
k, l, m und n sind unabhängig voneinander 0 bis 20. Bevorzugt sind l und m im Bereich von 0 bis 10, besonders bevorzugt von 0 bis 5, ganz besonders bevorzugt von 0 bis 3 und insbesondere bevorzugt 0 bis 2. Bevorzugt haben k und n den selben Wert, z. B. im Bereich von 0 bis 10, bevorzugt von 0 bis 5, ganz besonders bevorzugt von 1 bis 3, insbesondere haben k und n den Wert 1.

Die Reste R¹ bis R⁸ können im einzelnen die folgende Bedeutung haben:
R¹ und R² sowie R³ und R⁵ können unabhängig voneinander Wasserstoff, C₁-C₂₀-Alkyl, C₁-C₂₀-Alkylcarbonyl, C₂-C₂₀-Alkenyl, C₂-C₂₀-Alkenylcarbonyl, C₂-C₂₀-Alkinyl, C₂-C₂₀-Alkinylcarbonyl, C₃-C₁₅-Cycloalkyl, C₅-C₁₅-Cycloalkylcarbonyl, Aryl, Arylcarbonyl oder Heterocyclen sein,
R⁴ und R⁶ sowie R⁷ und R⁸ können unabhängig voneinander C₁-C₂₀-Alkyl, C₁-C₂₀-Alkylcarbonyl, C₂-C₂₀-Alkenyl, C₂-C₂₀-Alkenylcarbonyl, C₂-C₂₀-Alkinyl, C₂-C₂₀-Alkinylcarbonyl, C₃-C₁₅-Cycloalkyl, C₅-C₁₅-Cycloalkylcarbonyl, Aryl, Arylcarbonyl oder Heterocyclen sein,
wobei
a) im Falle der genannten aliphatischen Substituenten die Reste R³ und R⁴ beziehungsweise R⁵ und R⁶ auch miteinander verbunden sein können und so gemeinsam einen drei- bis achtgliedrigen, bevorzugt einen fünf- bis siebengliedrigen und besonders bevorzugt einen fünf- bis sechsgliedrigen Ring bilden können,
b) die genannten aliphatischen Substituenten geradkettig oder verzweigt sein können,
c) die Substituenten jeweils an beliebiger Position durch ein oder mehrere Heteroatome unterbrochen sein können, wobei die Anzahl dieser Heteroatome nicht mehr als 10 beträgt, bevorzugt nicht mehr als 8, besonders bevorzugt nicht mehr als 5 und insbesondere nicht mehr als 3, und/oder
d) die Substituenten jeweils an beliebiger Position, allerdings nicht mehr als fünfmal, bevorzugt nicht mehr als viermal und besonders bevorzugt nicht mehr als dreimal durch Alkyl, Alkyloxy, Alkyloxycarbonyl, Aryl, Aryloxy, Aryloxycarbonyl, Hydroxycarbonyl, Aminocarbonyl, Heterocyclen, Heteroatome oder Halogenatome substituiert sein können, wobei diese ebenfalls maximal zweimal, bevorzugt maximal einmal mit den genannten Gruppen substituiert sein können.

R⁹ bis R¹³ sind gegebenenfalls unabhängig voneinander Wasserstoff oder C₁-C₂₀-Alkyl.

Im einzelnen haben die für die verschiedenen Reste R angegebenen Sammelbegriffe folgende Bedeutung:

C₁-C₂₀-Alkyl: geradkettige oder verzweigte Kohlenwasserstoffreste mit bis zu 20 Kohlenstoffatomen, bevorzugt C₁-C₁₀-Alkyl wie Methyl, Ethyl, Propyl, Isopropyl, n-Butyl, sec-Butyl, tert.-Butyl, 1,1-Dimethylethyl, Pentyl, 2-Methylbutyl, 1,1-Dimethylpropyl, 1,2-Dimethylpropyl, 2,2-Dimethylpropyl, 1-Ethylpropyl, Hexyl, 2-Methylpentyl, 3-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 2-Ethylbutyl, 1,1,2-Trimethylpropyl, 1,2,2-Trimethylpropyl, 1-Ethyl-1-methylpropyl, 1-Ethyl-2-methylpropyl, Heptyl, Octyl, 2-Ethylhexyl, 2,4,4-Trimethylpentyl, 1,1,3,3-Tetramethylbutyl, Nonyl und Decyl sowie deren Isomere.

C₁-C₂₀-Alkylcarbonyl: eine geradkettige oder verzweigte Alkylgruppe mit 1 bis 20 Kohlenstoffatomen (wie vorstehend genannt), welche über eine Carbonylgruppe (-CO-) an gebunden ist, bevorzugt C₁₋C₁₀-Alkylcarbonyl wie beispielsweise Formyl, Acetyl, n-oder iso-Propionyl, n-, iso-, sec- oder tert.-Butanoyl, n-iso-, sec- oder tert.-Pentanoyl, n- oder iso-Nonanoyl, n-Dodecanoyl.

C₂-C₂₀-Alkenyl: ungesättigte, geradkettige oder verzweigte Kohlenwasserstoffreste mit 2 bis 20 Kohlenstoffatomen und einer Doppelbindung in einer beliebigen Position, bevorzugt C₂-C₁₀-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl, sowie die Isomere von Heptenyl, Octenyl, Nonenyl und Decenyl.

C₂-C₂₀-Alkenylcarbonyl: ungesättigte, geradkettige oder verzweigte Kohlenwasserstoffreste mit 2 bis 20 Kohlenstoffatomen und einer Doppelbindung in einer beliebigen Position (wie vorstehend genannt), welche über eine Carbonylgruppe (-CO-) an gebunden sind, bevorzugt C₂-C₁₀-Alkylcarbonyl wie beispielsweise Ethenoyl, Propenoyl, Butenoyl, Pentenoyl, Nonenoyl sowie deren Isomere.

C₂-C₂₀-Alkinyl: geradkettige oder verzweigte Kohlenwasserstoffgruppen mit 2 bis 20 Kohlenstoffatomen und einer Dreifachbindung in einer beliebigen Position, bevorzugt C₂-C₁₀-Alkinyl wie Ethinyl 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl sowie die Isomere von Heptinyl, Octinyl, Noninyl, Decinyl.

C₂-C₂₀-Alkinylcarbonyl ungesättigte, geradkettige oder verzweigte Kohlenwasserstoffreste mit 2 bis 20 Kohlenstoffatomen und einer Dreifachbindung in einer beliebigen Position (wie vorstehend genannt), welche über eine Carbonylgruppe (-CO-) an gebunden sind, bevorzugt C₂-C₁₀-Alkinylcarbonyl wie beispielsweise Propinoyl, Butinoyl, Pentinoyl, Noninoyl, Decinoyl sowie deren Isomere.

C₃-C₁₅-Cycloalkyl: monocyclische, gesättigte Kohlenwasserstoffgruppen mit 3 bis zu 15 Kohlenstoffringgliedern, bevorzugt C₃-C₈-Cycloalkyl wie Cyclopropyl, Cyclobutyl, Cyclopentyl, Cyclohexyl, Cycloheptyl und Cyclooctyl sowie ein gesättigtes oder ungesättigte cyclisches System wie z. B. Norbornyl oder Norbenyl.

C₃-C₁₅-Cycloalkylcarbonyl: monocyclische, gesättigte Kohlenwasserstoffgruppen mit 3 bis 15 Kohlenstoffringgliedern (wie vorstehend genannt), welche über eine Carbonylgruppe (-CO-) an sind, bevorzugt C₃-C₈-Cycloalkylcarbonyl.

Aryl: ein ein- bis dreikerniges aromatisches Ringsystem enthaltend 6 bis 14 Kohlenstoffringglieder, z. B. Phenyl, Naphthyl und Anthracenyl, bevorzugt ein ein- bis zweikerniges, besonders bevorzugt ein einkerniges aromatisches Ringsystem.

Arylcarbonyl: bevorzugt ein ein- bis dreikerniges aromatisches Ringsystem (wie vorstehend genannt), welches über eine Carbonylgruppe (-CO-) an gebunden ist, wie z. B. Benzoyl, bevorzugt ein ein- bis zweikerniges, besonders bevorzugt ein einkerniges arormatisches Ringsystem.

Heterocyclen: fünf- bis zwölfgliedrige, bevorzugt fünf- bis neungliedrige, besonders bevorzugt fünf- bis sechsgliedrige Sauerstoff-, Stickstoff- und/oder Schwefelatome aufweisender gegebenenfalls mehrere Ringe aufweisendes Ringsystem wie beispielsweise Furyl, Thiophenyl, Pyrryl, Pyridyl, Indolyl, Benzoxazolyl, Dioxolyl, Dioxyl, Benzimidazolyl, Benzthiazolyl, Dimethylpyridyl, Methylchinolyl, Dimethylpyrryl, Methoxyfuryl, Dimethoxypyridyl, Difluorpyridyl, Methylthiophenyl, Isopropylthiophenyl oder tert.-Butylthiophenyl.

Die im einzelnen aufgeführten Substituenten können wie zuvor beschrieben jeweils an beliebiger Position durch ein oder mehrere Heteroatome unterbrochen sein, wobei die Anzahl dieser Heteroatome nicht mehr als 10, bevorzugt nicht mehr als 8, ganz besonders bevorzugt nicht mehr als 5 und insbesondere nicht mehr als 3 beträgt, und/oder jeweils an beliebiger Position, allerdings nicht mehr als fünfmal, bevorzugt nicht mehr als viermal und besonders bevorzugt nicht mehr als dreimal, durch Alkyl, Alkyloxy, Alkyloxycarbonyl, Aryl, Aryloxy, Aryloxycarbonyl, Hydroxycarbonyl, Aminocarbonyl, Heterocyclen, Heteroatome oder Halogenatome substituiert sein können, wobei diese ebenfalls maximal zweimal, bevorzugt maximal einmal mit den genannten Gruppen substituiert sein können.

Die in dieser Gruppe genannten Verbindungsklassen Alkyl, Aryl und Heterocyclen haben die zuvor genannte Bedeutung.

Heteroatome sind Sauerstoff, Stickstoff, Schwefel oder Phosphor.

Alkyloxy bedeutet eine geradkettige oder verzweigte Alkylgruppe mit 1 bis 20 Kohlenstoffatomen (wie vorstehend genannt), welche über ein Sauerstoffatom (-O-) an gebunden sind, bevorzugt C₁-C₁₀-Alkyloxy wie beispislweise Methoxy, Ethoxy, Propoxy.

Alkoxycarbonyl ist eine Alkoxygruppe mit 1 bis 20 Kohlenstoffatomen (wie vorstehend genannt), welche über eine Carbonylgruppe (-CO-) an gebunden ist, bevorzugt C₁-C₁₀-Alkyloxycarbonyl.

Aryloxy ist ein ein- bis dreikerniges aromatisches Ringsystem (wie vorstehend genannt), welches über ein Sauerstoffatom (-O-) an gebunden ist, bevorzugt ein ein- bis zweikerniges, besonders bevorzugt ein einkerniges aromatisches Ringsystem.

Aryloxycarbonyl ist eine ein- bis dreikernige Aryloxygruppe (wie vorstehend genannt), welche über eine Carbonylgruppe (-CO-) an gebunden ist, bevorzugt ein ein- bsi zweikerniges, besonders bevorzugt ein einkerniges Aryloxycarbonyl.

Halogenatome sind Fluor, Chlor, Brom und lod.

Bevorzugt sind die Reste R¹ und R² gleich und sind Wasserstoff oder C₁-C₂₀-Alkyl, besonders bevorzugt Wasserstoff oder C₁-C₁₀-Alkyl, ganz besonders bevorzugt Wasserstoff oder C₁-C₆-Alkyl.

Bevorzugt sind die Reste R³ und R⁵ gleich und sind Wasserstoff, C₁-C₂₀-Alkyl oder C₁-C₂₀-Alkylcarbonyl, besonders bevorzugt Wasserstoff, C₁-C₁₀-Alkyl oder C₁-C₁₀-Alkylcarbonyl, ganz besonders bevorzugt Wasserstoff, C₁-C₆-Alkyl oder C₁-C₆-Alkylcarbonyl.

Bevorzugt sind die Reste R⁴ und R⁶ beziehungsweise R⁷ und R⁸ gleich und sind C₁-C₂₀-Alkyl, C₁-C₂₀-Alkylcarbonyl, Aryl, C₂-C₂₀-Alkenyl, C₂-C₂₀-Alkenylcarbonyl, C₂-C₂₀-Alkinyl oder C₂-C₂₀-Alkinylcarbonyl. Wie zuvor beschrieben können die Reste jeweils an beliebiger Position durch ein oder mehrere Heteroatome unterbrochen sein, wobei die Anzahl dieser Heteroatome nicht mehr als 10, bevorzugt nicht mehr als 8, ganz besonders bevorzugt nicht mehr als 5 und insbesondere nicht mehr als 3 beträgt, und/oder jeweils an beliebiger Position, allerdings nicht mehr als fünfmal, bevorzugt nicht mehr als viermal und besonders bevorzugt nicht mehr als dreimal, durch Alkyl, Alkyloxy, Alkyloxycarbonyl, Aryl, Aryloxy, Aryloxycarbonyl, Hydroxycarbonyl, Aminocarbonyl, Heterocyclen, Heteroatome oder Halogenatome substituiert sein können, wobei diese ebenfalls maximal zweimal, bevorzugt maximal einmal mit den genannten Gruppen substituiert sein können.

Besonders bevorzugte Reste R⁴ und R⁶ beziehungsweise R⁷ und R⁸ sind ausgewählt aus Phenyl, Benzyl, p-Methoxyphenyl, o-, m- oder p- Hydroxyphenyl, 1-Hydroxyhexyl, Methyl, Ethyl, Propyl, Butyl, Ethylenglykol, Diethylenglykol, Triethylenglykol, Ethoxylat mit 4 bis 10 EO-Einheiten, Ethylendiamin, Diethylentriamin, Triethylentetraamin und Aminosäuren wie beispielsweise Alanin, Valin, Leucin, Isoleucin, Prolin, Tryptophan, Phenylalanin, Methionin, Glycin, Serin, Tyrosin, Threonin, Cystein, Asparagin, Glutamin, Asparagin- oder Glutaminsäure, Lysin, Arginin oder Histidin.

Beispielsweise können R⁴ und R⁶ beziehungsweise R⁷ und R⁸ die in Tabelle 1 genannten Reste sein.

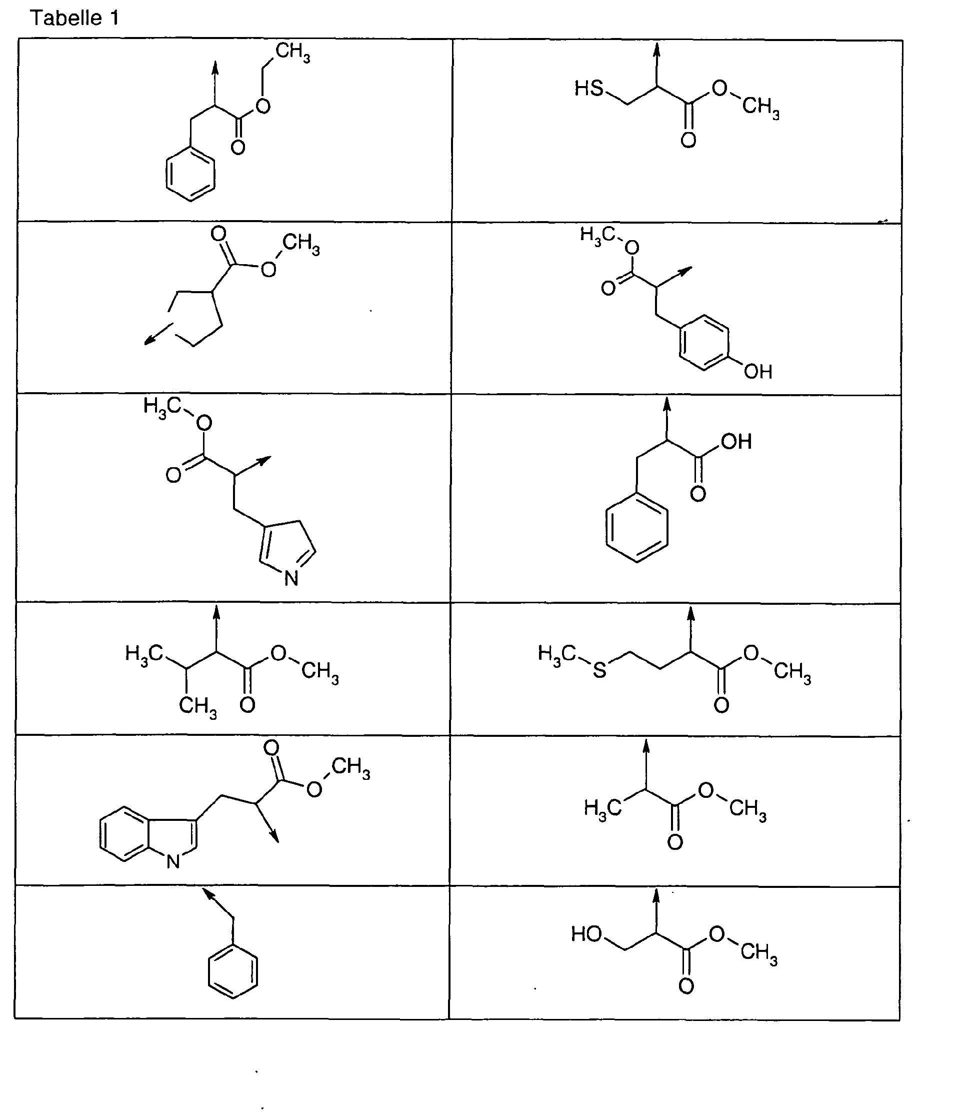

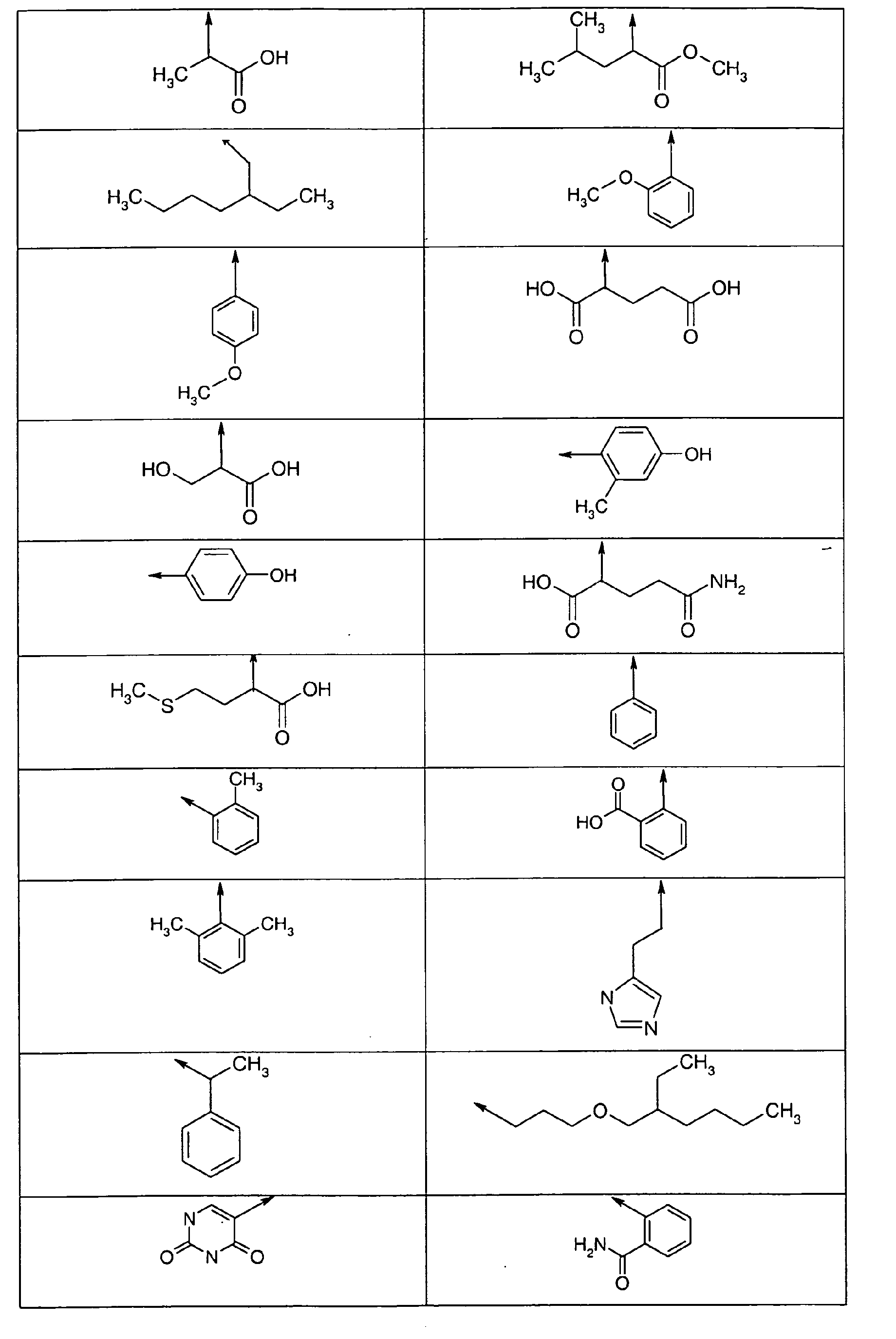

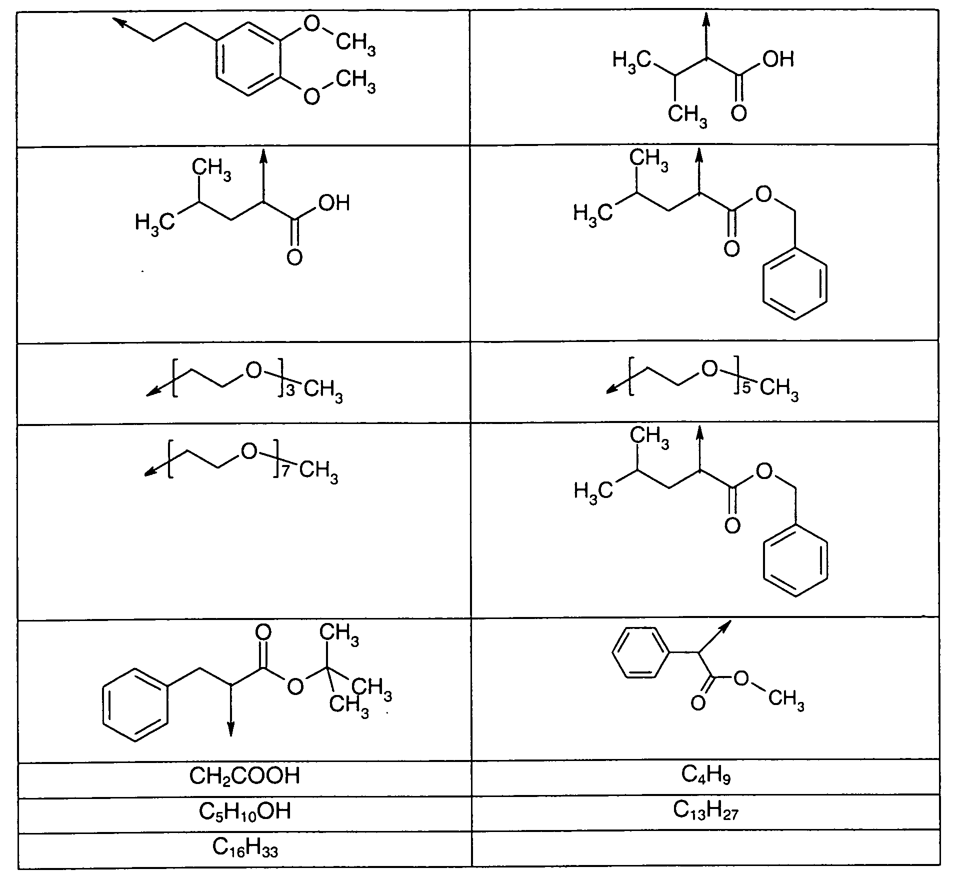

Selbstverständlich sind alle Kombinationen der genannten Substituenten R und X mit den genannten Zahlen für k, l, m und n möglich.

In Tabelle 2 sind die bevorzugten Individuen der Formel (I) zusammengefasst.

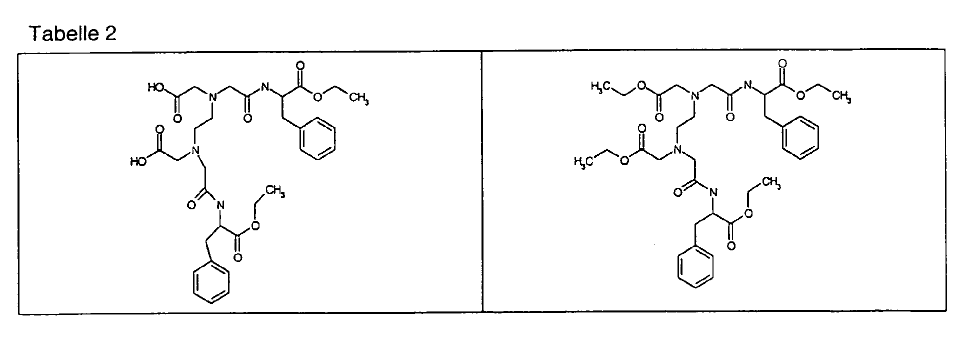

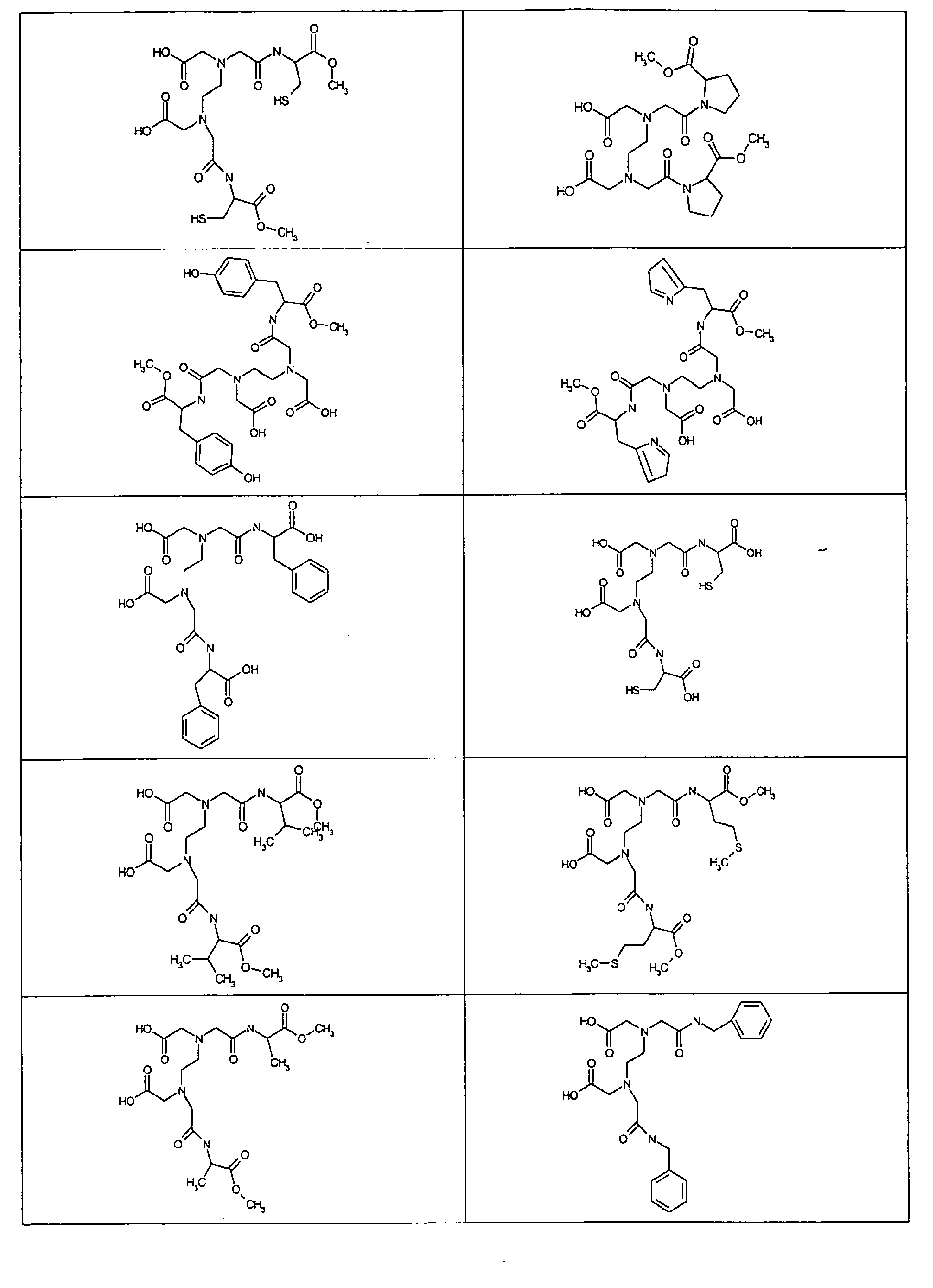

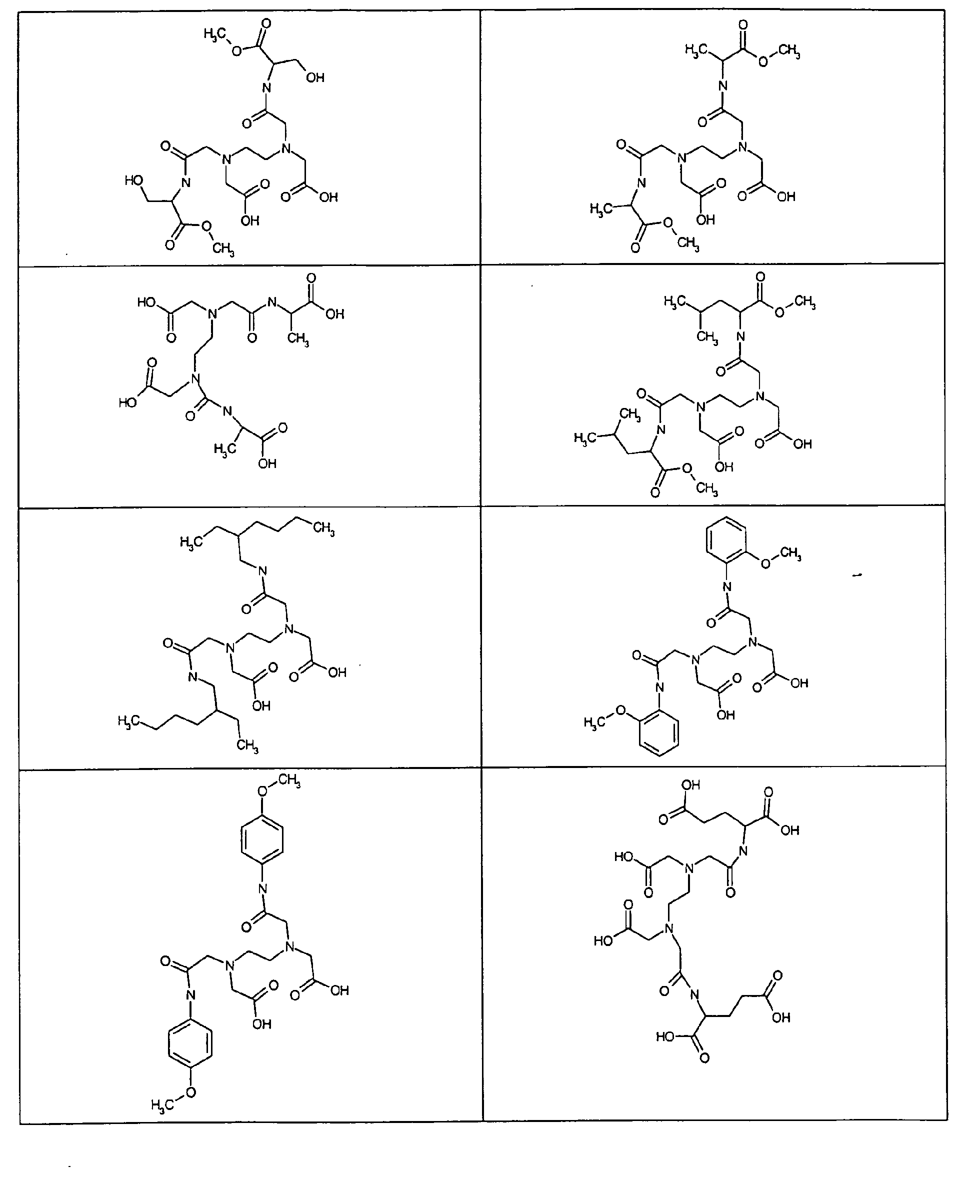

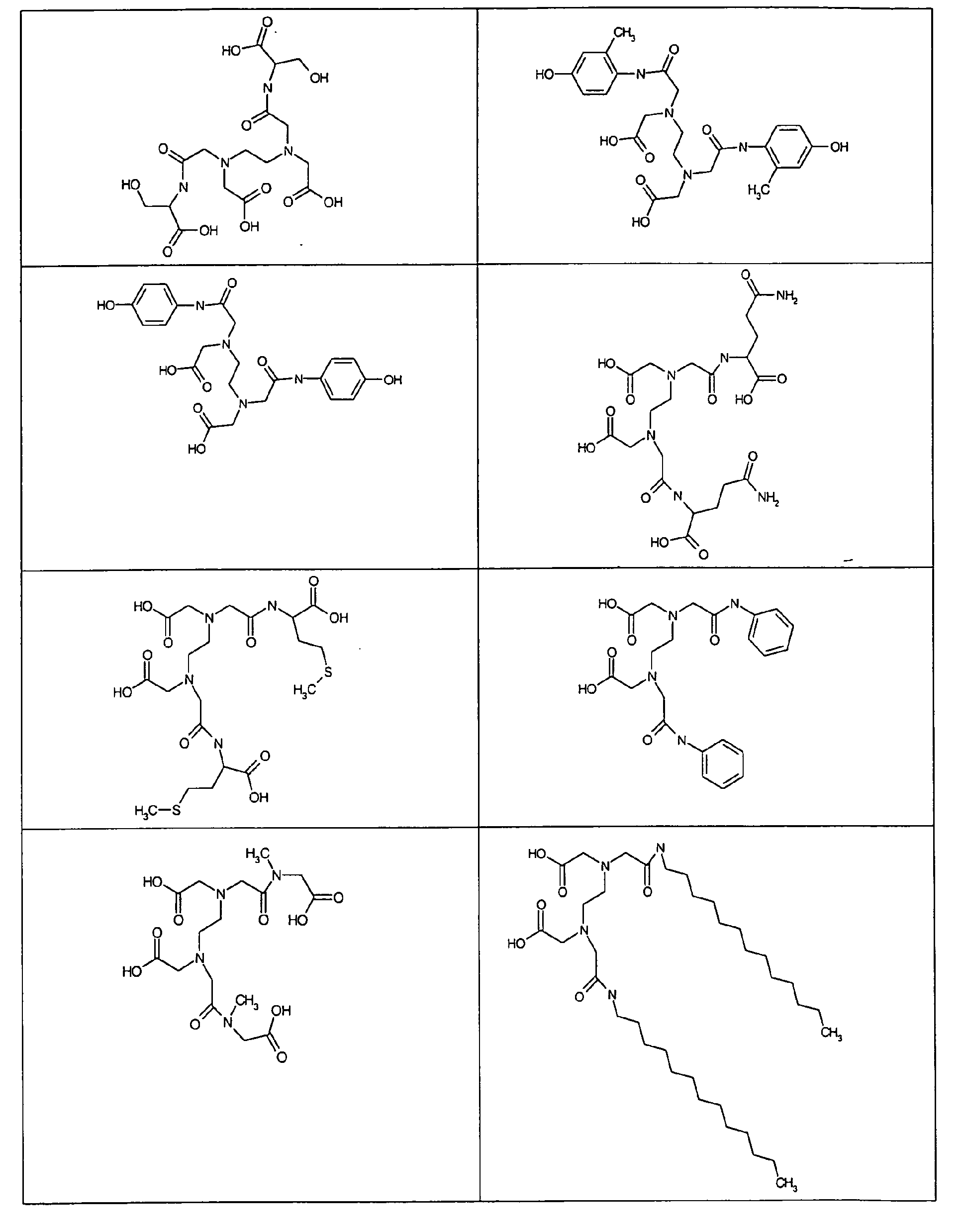

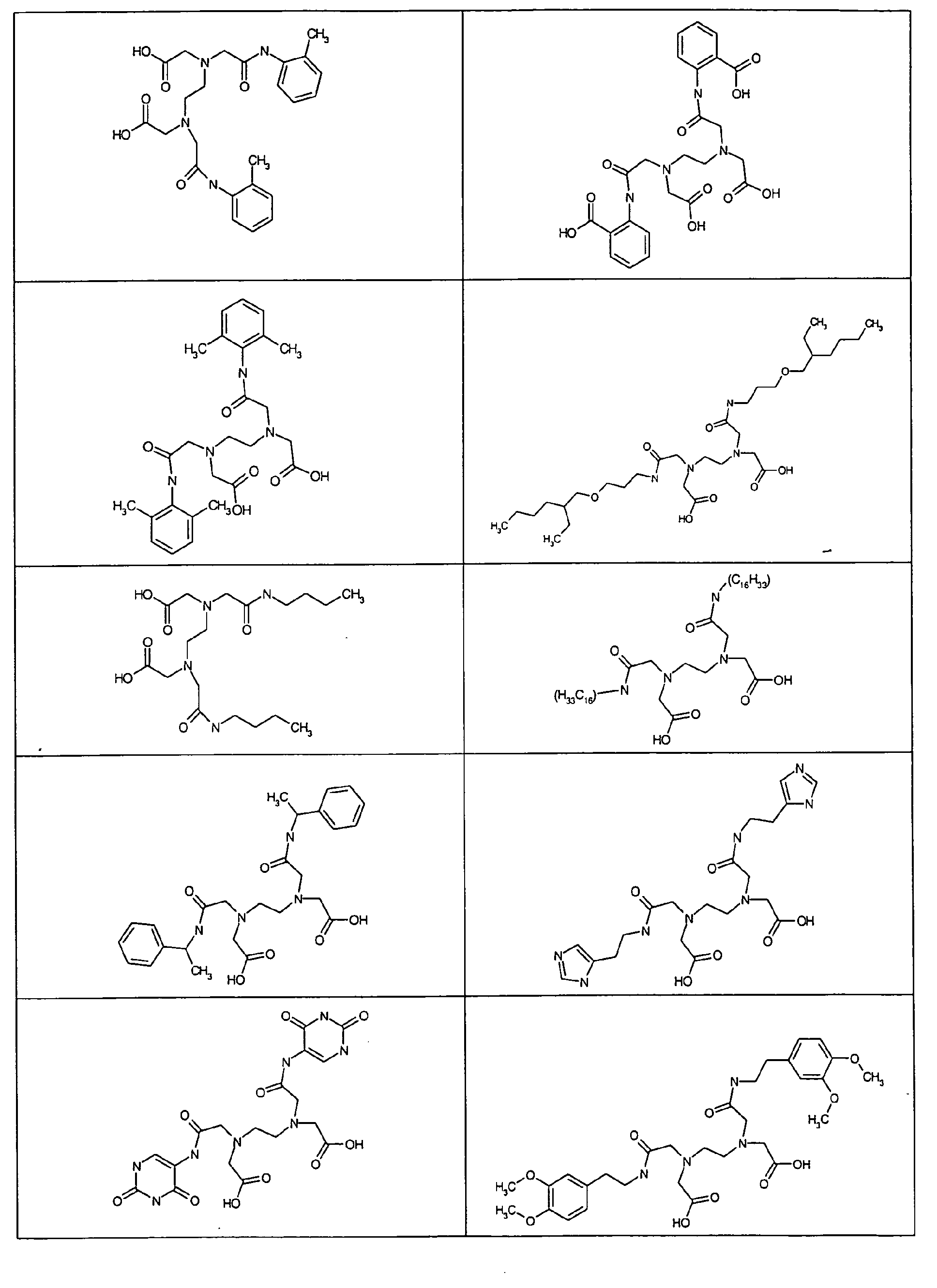

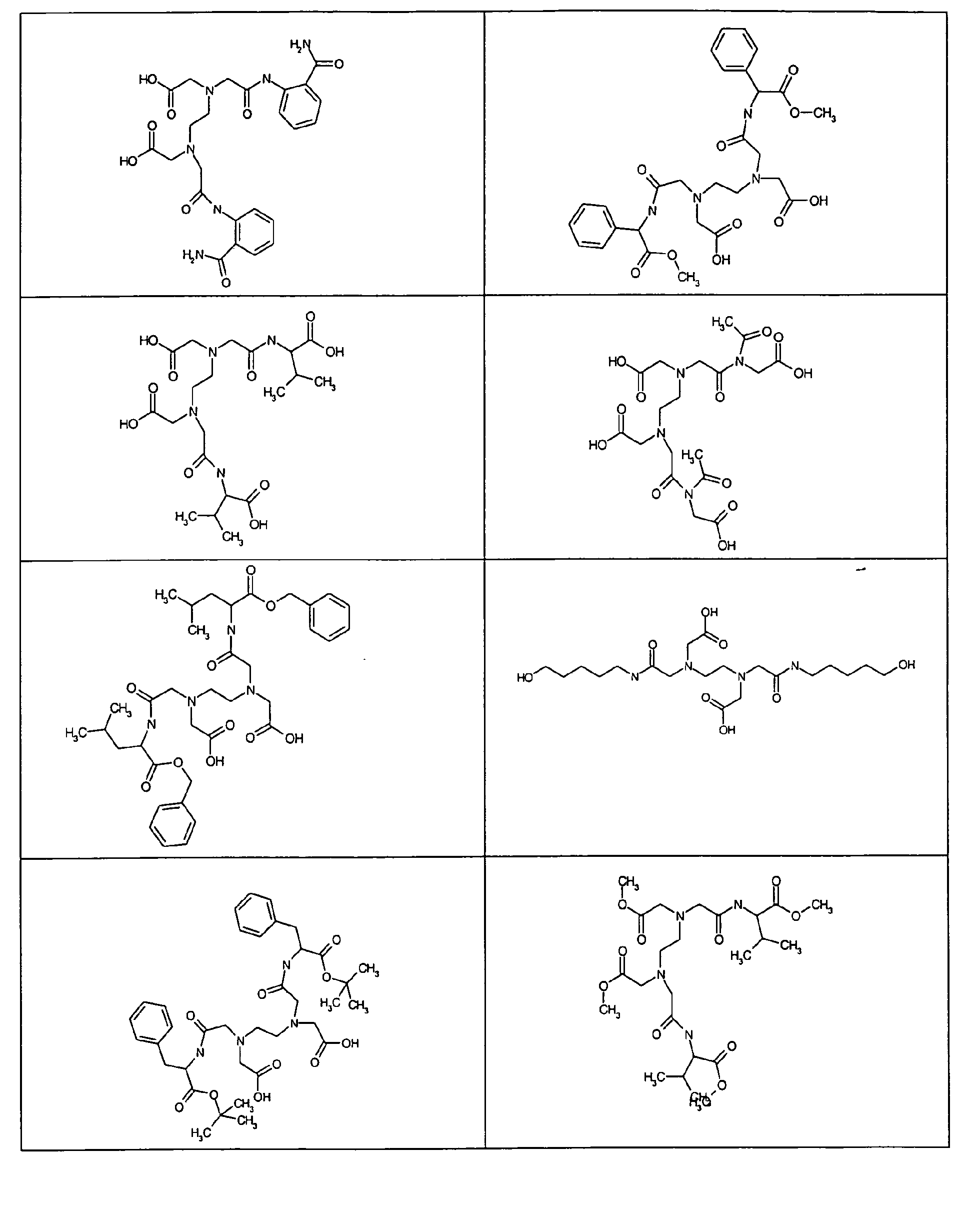

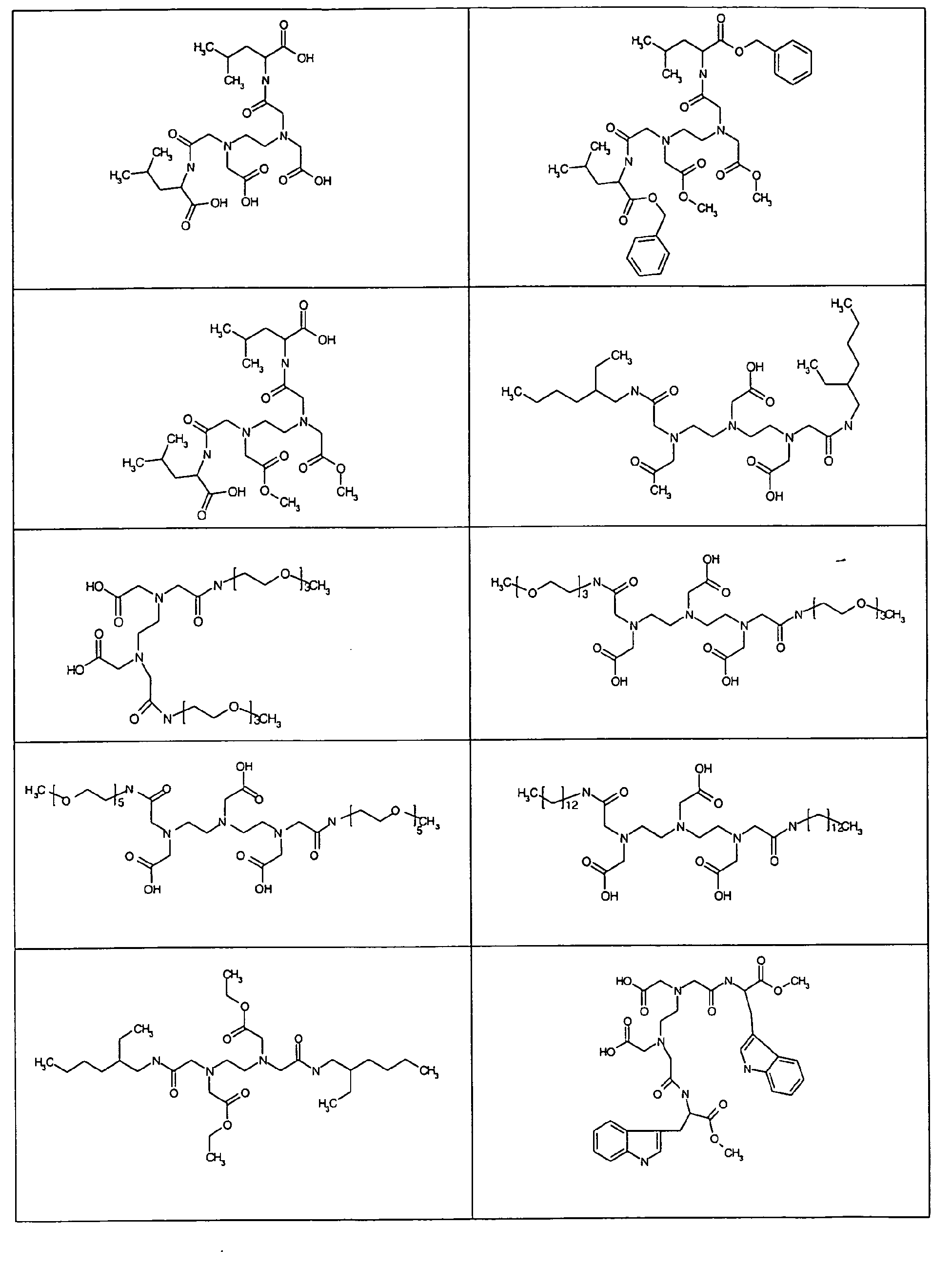

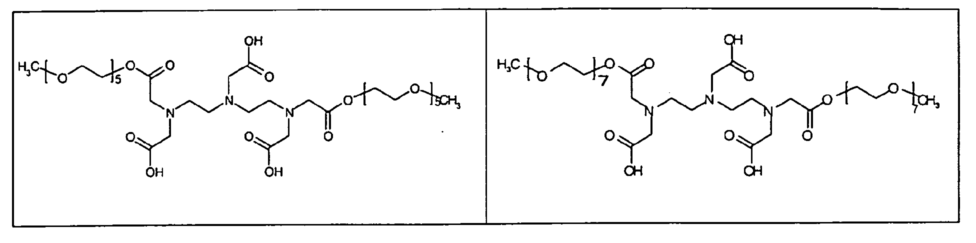

Die Herstellung dieser Verbindungen erfolgt beispielsweise analog der in DE-A 101 05 014 und CH-A 569 405 beschriebenen Synthesewege. Die darin offenbarten Verbindungen werden als Metallkomplexe bei Metallerscheinungen bzw. als Kontrastmittel in der Diagnostik verwendet. Ein genereller Syntheseweg wird in Bioorganic Medicinal Chemistry Letters 2001, 11, 2573 offenbart.

Üblicherweise werden die Radikalfänger einzeln oder als Gemisch eingesetzt, wobei bevorzugt nicht mehr als fünf, besonders bevorzugt nicht mehr als vier und ganz besonders bevorzugt nicht mehr als drei der zuvor genannten Radikalfänger eingesetzt werden.

Die Menge, in der die erfindungsgemäßen Radikalfänger eingesetzt werden, um eine stabilisierende Wirkung auf die polymerisationsfähige Verbindung auszuüben, sind im Rahmen fachüblicher Versuche zu ermitteln.

Beispielsweise werden von 0,1 bis 1000 ppm eines Radikalfängers oder eines Radikalfängergemisches bezogen auf die polymerisationsfähige Verbindung eingesetzt, bevorzugt 1 bis 900 ppm, besonders bevorzugt 10 bis 800 ppm, ganz besonders bevorzugt 50 bis 700 ppm und insbesondere 100 bis 500 ppm.

Die Radikalfänger können erfindungsgemäß vorteilhaft zusammen mit mindestens einer anderen als Stabilisator und/oder Costabilisator bekannten Verbindung verwendet werden. Diese sind z. B. in der älteren deutschen Patentanmeldung mit dem Aktenzeichen 102 49 507.6 sowie in DE-A 102 58 329, DE-A 198 56 565 und in EP-A 765 856 beschrieben.

Als Costabilisatoren kommen sauerstoffhaltige Gase, phenolische Verbindungen, Chinone und Hydrochinone, N-Oxylverbindungen, aromatische Amine und Phenylendiamine, Imine, Sulfonamide, Oxime, Hydroxylamine, Harnstoffderivate, phosphorhaltige Verbindungen, schwefelhaltige Verbindungen, Komplexbildner auf Basis von Tetraazaannulen (TAA) und/oder Metallsalze, sowie gegebenenfalls Mischungen davon in Frage.

Sauerstoffhaltige Gase können beispielsweise solche Gase sein, die einen Sauerstoffgehalt zwischen 0,1 und 100 Vol-%, bevorzugt von 0,5 bis 50 Vol.-% und besonders bevorzugt von 1 bis 25 Vol.-% aufweisen. Beispielsweise können dies Stickstoffmonoxid, Stickstoffdioxid, Sauerstoff oder Distickstofftrioxid oder Luft sein. Diese können einzeln, in beliebigen Mischungen miteinander oder mit einem anderen Gas vermischt sein, beispielsweise Stickstoff, Edelgase, Wasserdampf, Kohlenstoffmonoxid, Kohlenstoffdioxid oder niedere Alkane, bevorzugt sind Luft oder Luft-Stickstoff-Gemische.

Phenolische Verbindungen sind z. B: Phenol, Alkylphenole, beispielsweise o-, m- oder p-Kresol (Methylphenol), 2-tert.-Butyl-4-methylphenol, 6-tert.-Butyl-2,4-dimethylphenol, 2,6-Di-tert.-butyl-4-methylphenol, 2-tert.-Butylphenol, 4-tert.-Butylphenol, 2,4-Di-tert.butylphenol, 2-Methyl-4-tert.-butylphenol, 4-tert.-Butyl-2,6-dimethylphenol, oder 2,2'-Methylen-bis-(6-tert.-butyl-4-methylphenol), 4,4'-Oxydiphenyl, 3,4-Methylendioxydiphenol (Sesamol), 3,4-Dimethylphenol, Brenzcatechin (1,2-Dihydroxybenzol), 2-(1'-Methylcyclohex-1'-yl)-4,6-dimethylphenol, 2- oder 4-(1'-Phenyleth-1'-yl)phenol, 2-tert.-Butyl-6-methylphenol, 2,4,6-Tris-tert.-butylphenol, 2,6-Di-tert.-butylphenol, Nonylphenol [CAS-Nr. 11066-49-2], Octylphenol [CAS-Nr. 140-66-9], 2,6-Dimethylphenol, Bisphenol A, Bisphenol B, Bisphenol C, Bisphenol F, Bisphenol S, 3,3',5,5'-Tetrabromobisphenol A, 2,6-Di-tert.-butyl-p-kresol, Koresin® der BASF Aktiengesellschaft, 3,5-Di-tert.-butyl-4-hydroxybenzoesäuremethylester, 4-tert.-Butylbrenzcatechin, 2-Hydroxybenzylalkohol, 2-Methoxy-4-methylphenol, 2,3,6-Trimethylphenol, 2,4,5-Trimethylphenol, 2,4,6-Trimethylphenol, 2-lsopropylphenol, 4-Isopropylphenol, 6-Isopropyl-m-kresol, n-Octadecyl-β-(3,5-di-tert.-butyl-4-hydroxyphenyl)propionat, 1,1,3-Tris-(2-methyl-4-hydroxy-5-tert.-butylphenyl)butan, 1,3,5-Trimethyl-2,4,6-tris-(3,5-di-tert.-butyl-4-hydroxybenzyl)benzol, 1,3,5,-Tris-(3,5-ditert.-butyl-4-hydroxybenzyl)isocyanurat, 1,3,5,-Tris-(3,5-di-tert.-butyl-4-hydroxyphenyl)propionyloxyethyl-isocyanurat, 1,3,5-Tris-(2,6-dimethyl-3-hydroxy-4-tert.-butylbenzyl)isocyanurat oder Pentaerythrit-tetrakis-[β-(3,5,-di-tert.-butyl-4-hydroxyphenyl)propionat], 2,6-Di-tert.-butyl-4-dimethylaminomethylphenol, 6-sek.-Butyl-2,4-dinitrophenol, Irganox® 565, 1010, 1076, 1141, 1192, 1222 und 1425 der Firma Ciba Spezialitätenchemie, 3-(3',5'-Di-tert.-butyl-4'hydroxyphenyl)propionsäureoctadecylester, 3-(3',5'-Di-tert.-butyl-4'hydroxyphenyl)propionsäurehexadecylester, 3-(3',5'-Di-tert.-butyl-4'hydroxyphenyl)propionsäureoctylester, 3-Thia-1,5-pentandiol-bis-[(3',5'-di-tert.-butyl-4'hydroxyphenyl)propionat], 4,8-Dioxa-1,11-undecandio)-bis-[(3',5'-di-tert.-buty)-4'hydroxyphenyl)propionat], 4,8-Dioxa-1,11-undecandioi-bis-[(3'-*tert*.-buty)-4'-hydroxy-5'methylphenyl)propionat], 1,9-Nonandiol-bis-[(3',5'-di-tert.-butyl-4'hydroxyphenyl)propionat], 1,7-Heptandiamin-bis[3-(3',5'-di-tert.-butyl-4'hydroxyphenyl)propionsäureamid], 1,1-Methandiamin-bis[3-(3',5'-di-tert.-butyl-4'hydroxyphenyl)propionsäureamid], 3-(3',5'-Di-tert.-butyl-4'hydroxyphenyl)propionsäurehydrazid, 3-(3',5'-Dimethyl-4'hydroxyphenyl)propionsäurehydrazid, Bis-(3-tert.-butyl-5-ethyl-2-hydroxyphen-1-yl)methan, Bis-(3,5-di-tert.-butyl-4-hydroxyphen-1-yl)methan, Bis-[3-(1'-methylcyclohex-1'-yl)-5-methyl-2-hydroxyphen-1-yl]methan, Bis-(3-tert.-butyl-2-hydroxy-5-methylphen-1-yl)methan, 1,1-Bis-(5-tert.-butyl-4-hydroxy-2-methylphen-1-yl)ethan, Bis-(5-tert.-butyl-4-hydroxy-2-methylphen-1-yl)sulfid, Bis-(3-tert.-butyl-2-hydroxy-5-methylphen-1-yl)sulfid, 1,1-Bis-(3,4-dimethyl-2-hydroxyphen-1-yl)-2-methylpropan, 1,1-Bis-(5-tert.butyl-3-methyl-2-hydroxyphen-1-yl)butan, 1,3,5-Tris-[1'-(3",5"-di-tert.-butyl-4"hydroxyphen-1"-yl)meth-1'-yl]-2,4,6-trimethylbenzol, 1,1,4-Tris-(5'-tert.-butyl-4'hydroxy-2'-methylphen-1 '-yl)butan und tert.-Butylcatechol, sowie Aminophenole, wie z. B. p-Aminophenol, Nitrosophenole, wie z. B. p-Nitrosophenol, p-Nitroso-o-kresol, Alkoxyphenole, beispielsweise 2-Methoxyphenol (Guajacol, Brenzcatechinmonomethylether), 2-Ethoxyphenol, 2-lsopropoxyphenol, 4-Methoxyphenol (Hydrochinonmonomethylether), Mono- oder Di-tert.-butyl-4-methoxyphenol, 3,5-Di-tert.-butyl-4-hydroxyanisol, 3-Hydroxy-4-methoxybenzylalkohol, 2,5-Dimethoxy-4-hydroxybenzylalkohol (Syringaalkohol), 4-Hydroxy-3-methoxybenzaldehyd (Vanillin), 4-Hydroxy-3-ethoxybenzaldehyd (Ethylvanillin), 3-Hydroxy-4-methoxybenzaldehyd (Isovanillin), 1-(4-Hydroxy-3-methoxyphenyl)ethanon (Acetovanillon), Eugenol, Dihydroeugenol, Isoeugenol, Tocopherole, wie z. B. α-, β-, γ-, δ- und ε-Tocopherol, Tocol, α-Tocopherolhydrochinon, sowie 2,3-Dihydro-2,2-dimethyl-7-hydroxybenzofuran (2,2-Dimethyl-7-hydroxycumaran).

Als Chinone und Hydrochinone sind beispielsweise geeignet Hydrochinon oder Hydrochinonmonomethylether (4-Methoxyphenol), Methylhydrochinon, 2,5-Di-tert.-Butylhydrochinon, 2-Methyl-p-hydrochinon, 2,3-Dimethylhydrochinon, Trimethylhydrochinon, 4-Methylbrenzcatechin, tert-Butylhydrochinon, 3-Methylbrenzcatechin, Benzochinon, 2-Methyl-p-hydrochinon, 2,3-Dimethylhydrochinon, Trimethylhydrochinon, tert-Butylhydrochinon, 4-Ethoxyphenol, 4-Butoxyphenol, Hydrochinonmonobenzylether, p-Phenoxyphenol, 2-Methylhydrochinon, Tetramethyl-p-benzochinon, Diethyl-1,4-cyclohexandion-2,5-dicarboxylat, Phenyl-p-benzochinon, 2,5-Dimethyl-3-benzyl-pbenzochinon, 2-Isopropyl-5-methyl-p-benzochinon (Thymochinon), 2,6-Diisopropyl-pbenzochinon, 2,5-Dimethyl-3-hydroxy-p-benzochinon, 2,5-Dihydroxy-p-benzochinon, Embelin, Tetrahydroxy-p-benzochinon, 2,5-Dimethoxy-1,4-benzochinon, 2-Amino-5-methyl-p-benzochinon, 2,5-Bisphenylamino-1,4-benzochinon, 5,8-Dihydroxy-1,4-naphthochinon, 2-Anilino-1,4-naphthochinon, Anthrachinon, N,N-Dimethylindoanilin, N,N-Diphenyl-p-benzochinondiimin, 1,4-Benzochinondioxim, Coerulignon, 3,3'-Di-tert.butyl-5,5'-dimethyldiphenochinon, p-Rosolsäure (Aurin), 2,6-Di-tert.-butyl-4-benzylidenbenzochinon, 2,5-Di-tert.-Amylhydrochinon.

Als N-Oxyle (Nitroxyl- oder N-Oxyl-Radikale, Verbindungen, die wenigstens eine >N-O•-Gruppe aufweisen) sind z. B. geeignet 4-Hydroxy-2,2,6,6-tetramethyl-piperidin-N-oxyl, 4-Oxo-2,2,6,6-tetramethyl-piperidin-N-oxyl, 4-Methoxy-2,2,6,6-tetramethylpiperidin-N-oxyl, 4-Acetoxy-2,2,6,6-tetramethyl-piperidin-N-oxyl, 2,2,6,6-Tetramethyl-piperidin-N-oxyl, Uvinul® 4040P der BASF Aktiengesellschaft, 4,4',4"-Tris-(2,2,6,6-tetramethyl-piperidin-N-oxyl)phosphit, 3-Oxo-2,2,5,5-tetramethyl-pyrrolidin-N-oxyl, 1-Oxyl-2,2,6,6-tetramethyl-4-methoxypiperidin, 1-Oxyl-2,2,6,6-tetramethyl-4-trimethylsilyloxypiperidin, 1-Oxyl-2,2,6,6-tetramethylpiperidin-4-yl-2-ethylhexanoat, 1-Oxyl-2,2,6,6-tetramethylpiperidin-4-yl-sebacat, 1-Oxyl-2,2,6,6-tetramethylpiperidin-4-ylstearat, 1-Oxyl-2,2,6,6-tetramethylpiperidin-4-yl-benzoat, 1-Oxyl-2,2,6,6-tetramethylpiperidin-4-yl-(4-tert-butyl)benzoat, Bis-(1-oxyl-2,2,6,6-tetramethylpiperidin-4-yl)succinat, Bis-(1-oxyl-2,2,6,6-tetramethylpiperidin-4-yl)adipat, 1,10-Dekandisäurebis-(1-oxyl-2,2,6,6-tetramethylpiperidin-4-yl)ester, Bis-(1-oxyl-2,2,6,6-tetramethylpiperidin-4-yl)n-butylmalonat, Bis-(1-oxyl-2,2,6,6-tetramethylpiperidin-4-yl)phthalat, Bis-(1-oxyl-2,2,6,6-tetramethylpiperidin-4-yl)isophthalat, Bis-(1-oxyl-2,2,6,6-tetramethylpiperidin-4-yl)terephthalat, Bis-(1-oxyl-2,2,6,6-tetramethylpiperidin-4-yl)hexahydroterephthalat, N,N'-Bis-(1-oxyl-2,2,6,6-tetramethylpiperidin-4-yl)adipinamid, N-(1-Oxyl-2,2,6,6-tetramethylpiperidin-4-yl)caprolactam, N-(1-Oxyl-2,2,6,6-tetramethylpiperidin-4-yl)dodecylsuccinimid, 2,4,6-Tris-[N-butyl-N-(1-oxyl-2,2,6,6-tetramethylpiperidin-4-yl]triazin, N,N'-Bis-(1-oxyl-2,2,6,6-tetramethylpiperidin-4-yl)-N,N'bis-formyl-1,6-diaminohexan, 4,4'-Ethylen-bis-(1-oxyl-2,2,6,6-tetramethylpiperazin-3-on).

Als aromatische Amine oder Phenylendiamine sind beispielsweise geeignet N,N-Diphenylamin, N-Nitroso-diphenylamin, Nitrosodiethylanilin, p-Phenylendiamin, N,N'--Dialkyl-p-phenylendiamin, wobei die Alkylreste gleich oder verschieden sein können und jeweils unabhängig voneinander aus 1 bis 4 Kohlenstoffatome bestehen und geradkettig oder verzweigt sein können, beispielsweise N,N'-Di-iso-butyl-pphenylendiamin, N,N'-Di-iso-propyl-p-phenylendiamin, Irganox® 5057 der Firma Ciba Spezialitätenchemie, N-Phenyl-p-phenylendiamin, N,N'-Diphenyl-p-phenylendiamin, N-Isopropyl-N-phenyl-p-phenylendiamin, N,N'-Di-sec.-butyl-p-phenylendiamin (Kerobit® BPD der BASF Aktiengesellschaft), N-Phenyl-N'-isopropyl-p-phenylendiamin (Vulkanox@ 4010 der Bayer AG), N-(1,3-Dimethylbutyl)-N'-phenyl-p-phenylendiamin, N-Phenyl-2-naphthylamin, Imoinodibenzyl, N,N'-Diphenylbenzidin, N-Phenyltetraanilin, Acridon, 3-Hydroxydiphenylamin, 4-Hydroxydiphenylamin.

Imine sind z. B. Methylethylimin, (2-Hydroxyphenyl)benzochinonimin, (2-Hydroxyphenyl)benzophenonimin, N,N-Dimethylindoanilin, Thionin (7-Amino-3-imino-3H-phenothiazin), Methylen violett (7-Dimethylamino-3-phenothiazinon).

Als Stabilisator wirksame Sulfonamide sind beispielsweise N-Methyl-4-toluolsulfonamid, N-tert.-Butyl-4-toluolsulfonamid, N-tert.-Butyl-N-oxyl-4-toluolsulfonamid, N,N'-Bis(4-sulfanilamid)piperidin, 3-{[5-(4-Aminobenzoyl)-2,4-dimethylbenzolsulfonyl]ethylamino}-4-methylbenzolsulfonsäure, wie in DE-A 102 58 329.

Oxime können beispielsweise Aldoxime, Ketoxime oder Amidoxime sein, wie beispielsweise in DE-A 101 39 767 beschrieben, bevorzugt Diethylketoxim, Acetonoxim, Methylethylketoxim, Cylcohexanonoxim, Benzaldehydoxim, Benzildioxim, Dimethylglyoxim, 2-Pyridinaldoxim, Salicylaldoxim, Phenyl-2-pyridylketoxim, 1,4-Benzochinondioxim, 2,3-Butandiondioxim, 2,3-Butandionmonooxim, 9-Fluorenonoxim, 4-tert.-Butyl-cyclohexanonoxim, N-Ethoxy-acetimidsäureethylester, 2,4-Dimethyl-3-pentanonoxim, Cyclododecanonoxim, 4-Heptanonoxim und Di-2-Furanylethandiondioxim oder andere aliphatische oder aromatische Oxime beziehungsweise deren Reaktionsprodukte mit Alkylübertragungsreagenzien, wie z. B. Alkylhalogenide, -triflate, -sulfonate, -tosylate, -carbonate, -sulfate, -phosphate oder dergleichen.

Hydroxylamine sind z. B. N,N-Diethylhydroxylamin und solche, die in der internationalen Anmeldung mit dem Aktenzeichen PCT/EP/03/03139 offenbart sind.

Als Harnstoffderivate sind beispielsweise geeignet Harnstoff oder Thioharnstoff.

Phosphorhaltige Verbindungen sind z. B. Triphenylphosphin, Triphenylphosphit, Hypophosphorige Säure, Trinonylphsophit, Triethylphosphit oder Diphenylisopropylphosphin.

Als schwefelhaltige Verbindungen sind beispielsweise geeignet Diphenylsulfid, Phe--nothiazin und schwefelhaltige Naturstoffe wie Cystein.

Komplexbildner auf Basis von Tetraazannulen (TAA) sind z. B. Diebenzotetraaza[14]annulene und Porphyrine wie sie in Chem. Soc. Rev. 1998, 27, 105-115 genannt werden.

Metallsalze sind z. B. Kupfer-, Mangan-, Cer-, Nickel-, Chrom-, -carbonat, -chlorid, - dithiocarbamat, -sulfat, -salicylat oder -acetat, -stearat, -ethylhexanoat.

Bevorzugte Costabilisatoren sind sauerstoffhaltige Gase, Phenothiazin, o-, m- oder p-Kresol (Methylphenol), 2-tert.-Butyl-4-methylphenol, 2,6-Di-tert.-butyl-4-methylphenol, 2-tert.-Butylphenol, 4-tert.-Butylphenol, 2,4-Di-tert.-butylphenol, Brenzcatechin (1,2-Dihydroxybenzol), 2,6-Di-tert.-butylphenol, 4-tert.-Butyl-2,6-dimethylphenol, Octylphenol [140-66-9], Nonylphenol [11066-49-2], 2,6-Dimethylphenol, 2,6-Di-tert.-butyl-pkresol, Bisphenol A, tert.-Butylcatechol, Hydrochinon, Hydrochinonmonomethylether oder Methylhydrochinon, 2,2,6,6-Tetramethyl-piperidin-N-oxyl, 4-Hydroxy-2,2,6,6-tetramethyl-piperidin-N-oxyl, 4-Oxo-2,2,6,6-tetramethyl-piperidin-N-oxyl sowie Mangan(II)acetat, Cer(III)carbonat, Cer(III)acetat oder Cer(III)ethylhexanoat, Cer(III)stearat sowie Gemische davon in unterschiedlicher Zusammensetzung.

Besonders bevorzugt sind Luft, Luft-Stickstoff-Gemische, Phenothiazin, o-, m- oder p-Kresol (Methylphenol), 2,6-Di-tert.-butyl-4-methylphenol, 4-tert.-Butylphenol, 4-tert.-Butyl-2,6-dimethylphenol, Octylphenol [140-66-9], Nonylphenol [11066-49-2], 2,6-Dimethylphenol, 2,6-Di-tert.-butyl-p-kresol, tert.-Butylcatechol, Hydrochinon, Hydrochinonmonomethylether oder Methylhydrochinon, 2,2,6,6-Tetramethyl-piperidin-N-oxyl, 4-Hydroxy-2,2,6,6-tetramethyl-piperidin-N-oxyl, 4-Oxo-2,2,6,6-tetramethyl-piperidin-N-oxyl sowie Cer(III)acetat oder Cer(III)ethylhexanoat und Gemische aus wenigstens zwei der genannten Komponenten.

Insbesondere bevorzugt sind Luft, Luft-Stickstoffgemische, Phenothiazin, Hydrochinon und Hydrochinonmonomethylether sowie 2,2,6,6-Tetramethyl-piperidin-N-oxyl, 4-Hydroxy-2,2,6,6-tetramethyl-piperidin-N-oxyl, 4-Oxo-2,2,6,6-tetramethyl-piperidin-N-oxyl, N,N'-Di-sec.-butyl-p-phenylendiamin, Cer(III)acetat oder Cer(III)ethylhexanoat und Gemische davon.

Die Art der Zugabe des erfindungsgemäßen Stabilisators und der gegebenenalls einzusetzenden Costabilisatoren ist nicht beschränkt. Der zugesetzte erfindungsgemäße Stabilisator kann jeweils einzeln oder als Gemisch mit weiteren erfindungsgemäßen Stabilisatoren und/oder mit vorgenannten Costabilisatoren zugesetzt werden, in flüssiger oder in gelöster Form in einem geeigneten Lösungsmittel, wobei dieses Lösungsmittel selber ein Stabilisator sein kann, wie z. B. in DE-A 102 00 583 beschrieben. Als Lösungsmittel geeignet sind weiterhin beispielsweise Stoffströme aus der Herstellung - der polymerisationsfähigen Verbindung. Dies können beispielsweise die reinen Produkte, d. h. die polymerisationsfähigen Verbindungen, in einer Reinheit von in der Regel 95 % oder mehr, bevorzugt 98 % oder mehr und besonders bevorzugt 99 % oder mehr verwendet werden, aber auch die zur Herstellung der polymerisationsfähigen Verbindungen eingesetzten Edukte, in einer Reinheit von 95 % oder mehr, bevorzugt 98 % oder mehr und besonders bevorzugt 99 % oder mehr, oder solche Stoffströme, die Edukte und/oder Produkte und/oder Zwischenprodukte und/oder Nebenprodukte enthalten.

Die Konzentration der eingesetzten Lösungen ist nur durch die Löslichkeit des Stabilisators/Stabilisatorgemisches in dem Lösungsmittel begrenzt, beispielsweise kann sie 0,1 - 50 Gew.-% betragen, bevorzugt 0,2 - 25 Gew.-%, besonders bevorzugt 0,3 - 10 Gew.-% und ganz besonders bevorzugt 0,5 - 5 Gew.-%.

Selbstverständlich können die erfindungsgemäßen Radikalfänger bzw. Radikalfängergemische auch als Schmelze eingesetzt werden, beispielsweise wenn der Schmelzpunkt unter 120 °C, bevorzugt unter 100 °C, besonders bevorzugt unter 80 °C und insbesondere unter 60 °C beträgt.

In einer weiteren bevorzugten Form werden die erfindungsgemäßen Radikalfänger bzw. Radikalfängergemische als Schmelze mit einem Phenol mit einem Schmelzpunkt unter 120 °C, bevorzugt unter 100 °C, besonders bevorzugt unter 80 °C und insbesondere unter 60 °C als Costabilisator eingesetzt. Besonders bevorzugt ist das Phenol ausgewählt unter p-Aminophenol, p-Nitrosophenol, 2-tert.-Butylphenol, 4-tert.-Butylphenol, 2,4-di-tert.-Butylphenol, 2-Methyl-4-tert.-Butylphenol, 4-tert.-Butyl-2,6-dimezhylphenol, Hydrochinon und Hydrochinonmonomethylether.
Wie zuvor beschrieben werden 0,1 bis 1000 ppm des erfindungsgemäßen Radikalfängers oder eines Gemisches von Radikalfängern bezogen auf die polymerisationsfähige Verbindung eingesetzt. Wird ein Gemisch von erfindungsgemäßen Radikalfängern mit Costabilisatoren verwendet, so werden 0,1 bis 5000 ppm, bevorzugt 1 bis 4000 ppm, besonders bevorzugt 5 bis 2500 ppm, besonders bevorzugt 10 bis 1000 ppm und insbesondere 50 bis 750 ppm bezogen auf die polymerisationsfähige Verbindung eingesetzt.

Wird ein Gemisch von mehreren Stabilisatoren bzw. Costabilisatoren verwendet, so können diese sowohl unabhängig voneinander an verschiedenen oder gleichen Dosierstellen zugeführt werden als auch unabhängig voneinander in unterschiedlichen Lösungsmitteln gelöst werden.

Erfindungsgemäß werden die Stabilisatoren/Stabilisatorgemische bevorzugt an solchen Stellen eingesetzt, an denen die polymerisationsfähige Verbindung, beispielsweise durch hohe Reinheit, hohe Verweilzeit und/oder hohe Temperatur, einer Polymeri--sationsgefahr ausgesetzt ist.

Dies können beispielsweise Absorptionseinheiten, Desorptionseinheiten, Rektifikationseinheiten, beispielsweise Destillationsapparate oder Rektifikationskolonnen, Verdampfer, beispielsweise Natur- oder Zwangsumlaufverdampfer, Kondensatoren oder Vakuumeinheiten sein.

Beispielsweise können die Stabilisatoren am Kopf einer Rektifikationseinheit eindosiert werden, z. B. in den Kopf der Rektifikationseinheit, Entnahmeeinbauten oder über die trennwirksamen Einbauten, wie z. B. Böden, Packungen, Wellenbrecher oder Schüttungen, gesprüht oder gedüst oder zusammen mit dem Rücklauf, in einen Kondensator eindosiert werden, z. B. eingesprüht, so dass der Kondensatorkopf und/oder die Kühlflächen benetzt sind, oder in einer Vakuumeinheit, wie in der EP-A 1 057 804 beschrieben oder als Sperrflüssigkeit in einer Flüssigkeitsringpumpe, wie in DE-A 101 43 565 beschrieben.

Die erfindungemäß einzusetzenden Radikalfänger können sowohl als Lager- als auch als Transportstabilisator verwendet werden, d. h. zur Stabilisierung der reinen polymerisationsfähigen Verbindungen.

Weiterhin sind Gegenstand der Erfindung Stabilisatormischungen, enthaltend
i) wenigstens einen Radikalfänger, der mindestens zwei Glycineinheiten enthält, mit der Maßgabe, dass der Radikalfänger nicht ausgewählt ist aus Ethylendiamintetraessigsäure (EDTA), Diethylentriaminpentaessigsäure (DTPA), trans-1,2-Cyclohexandiamintetraessigsäure (CYDTA) und deren Alkali- und Erdalkalisalze und
ii) wenigstens einen weiteren Stabilisator oder Costabilisator.

Radikalfänger mit mindestens zwei Glycineinheiten i) sind darin Verbindungen, die bevorzugt wenigstens eine Amid- und/oder Estereinheit enthalten, besonders bevorzugt Radikalfänger der Formel (I). Stabilisatormischungen mit den zuvor genannten bevorzugten erfindungsgemäßen Radikalfängern sind selbstverständlich ebenfalls Gegenstand der Erfindung.

Dabei sind Kombinationen aller zuvor genannter Komponenten möglich.

Stabilisatoren oder Costabilisatoren sind die oben angeführten sauerstoffhaltigen Gase, phenolischen Verbindungen, Chinone und Hydrochinone, N-Oxylverbindungen, aromatischen Amine und Phenylendiamine, Imine, Sulfonamide, Oxime, Hydroxylamine, Harnstoffderivate, phosphorhaltigen und/oder schwefelhaltigen Verbindungen, Komplexbildner auf TAA-Basis und/oder Metallsalze.

Bevorzugt sind Stabilisatormischungen aus dem Radikalfänger und Phenothiazin, Radikalfänger/Hydrochinon, Radikalfänger/Hydrochinonmonomethylether, Radikalfänger/4-Hydroxy-2,2,6,6-tetramethyl-piperidin-N-oxyl, Radikalfänger/4-Oxo-2,2,6,6-tetramethyl-piperidin-N-oxyl, Radikalfänger/2,2,6,6-tetramethyl-piperidin-N-oxyl, Radikalfänger/Phenothiazin/Hydrochinonmonomethylether, Radikalfänger/Phenothiazin/4-Hydroxy-2,2,6,6-tetramethyl-piperidin-N-oxyl oder Radikalfänger/Hydrochinonmonomethylether/4-Hydroxy-2,2,6,6-tetramethyl-piperidin-N-oxyl sowie gegebenenfalls jeweils wenigstens eines der genannten Cer-Salze, jeweils in Anoder Abwesenheit eines sauerstoffhaltigen Gases, bevorzugt in Anwesenheit.

Die erfindungsgemäßen Stabilisatormischungen enthalten die Komponenten i) und ii) in Gewichtsverhältnissen i) : ii) zwischen 1:100 bis 100:1, bevorzugt 1:50 bis 50:1, besonders bevorzugt 1:10 bis 10:1 und insbesondere 1:5 bis 5:1.

Gegenstand der Erfindung sind weiterhin Stoffmischungen, die die vorgenannten erfindungsgemäßen Stabilisatormischungen und wenigstens eine polymerisationsfähige Verbindung enthalten. Dabei sind alle Kombinationen von erfindungsgemäßen Stabilisatormischungen mit polymerisationsfähigen Verbindungen möglich.

Die Verwendung der Stoffmischungen, die die vorgenannten Stabilisatormischungen enthalten, zur Stabilisierung polymerisationsfähiger.Verbindungen gegen Polymerisation bei der Aufarbeitung, Lagerung und/oder Transport ist ebenfalls Gegenstand der Erfindung.

Polymerisationsfähige Verbindungen im Sinne der vorliegenden Erfindung sind solche mit mindestens einer ethylenisch ungesättigten Gruppe. Diese sind ausgewählt aus mono-, di- oder triethylenisch ungesättigten C₃-C₈-Carbonsäuren, C₁-C₂₀-Estern, - Amiden, -Nitrilen und -Anhydriden dieser mono-, di- oder triethylenisch ungesättigten C₃-C₈-Carbonsäuren, Vinylestern von bis zu 20 C-Atome enthaltenden Carbonsäuren, Vinylethern von 1 bis 10 C-Atome enthaltenden Alkoholen, Vinylaromaten und - heteroaromaten mit bis zu 20 C-Atomen, Vinyllactamen mit 3 bis 10 C-Atomen im Ring, offenkettigen N-Vinylamidverbindungen und N-Vinylaminverbindungen, Vinylhalogeniden, aliphatischen gegebenenfalls halogenierten Kohlenwasserstoffen mit 2 bis 8 C-Atomen und 1 oder 2 Doppelbindungen, Vinylidenen oder Mischungen dieser Monomeren.

Bevorzugte mono-, di- oder triethylenisch ungesättigte C₃-C₆-Carbonsäuren sind z. B. (Meth)acrylsäure, Dimethylacrylsäure, Ethacrylsäure, Citraconsäure, Methylenmalonsäure, Crotonsäure, Fumarsäure, Mesaconsäure, Itaconsäure, Maleinsäure sowie deren C₁-C₂₀-Alkylester, -Amide, -Nitrile, -Aldehyde und -Anhydride wie z. B. (Meth)acrylsäuremethylester, (Meth)acrylsäureethylester, (Meth)acrylsäure-nbutylester, (Meth)acrylsäure-n-propylester, (Meth)acrylsäure-iso-propylester, (Meth)acrylsäure-2-ethylhexylester, (Meth)acrylsäurearylester, , Maleinsäuremonomethylester, Maleinsäuredimethylester, Maleinsäuremonoethylester, Maleinsäurediethylester, Alkylenglykol(meth)acrylate, (Meth)acrylamid, N-Dimethyl(meth)acrylamid, N-tert.-butyl(meth)acrylamid, (Meth)acrylnitril, (Meth)acrolein, (Meth)acrylsäureanhydrid, Itaconsäureanhydrid, Maleinsäureanhydrid sowie dessen Halbester. Kationische Monomere dieser Gruppe sind beispielsweise Dialkylaminoalkyl(meth)acrylate und Dialkylaminoalkyl(meth)acrylamide wie Dimethylaminomethyl(meth)acrylat, Dimethylaminoethyl(meth)acrylat Diethylaminoethyl(meth)acrylat sowie die Salze der zuletzt genannten Monomeren mit Carbonsäuren oder Mineralsäuren sowie die qauternierten Produkte.

Weitere Monomere dieser Gruppe sind z. B. auch Hydroxylgruppen enthaltende Monomere, insbesondere C₁-C₁₀-Hydroxyalkyl(meth)acrylate wie beispielsweise Hydroxyethyl(meth)acrylat, Hydroxypropyl(meth)acrylat, Hydroxybutyl(meth)acrylat, Hydroxyisobutyl(meth)acrylat.

Weitere Monomere dieser Gruppe sind Phenyloxyethylglykol-mono-(meth)acrylat, Glycidyl(meth)acrylat, Trimethylolpropantriacrylt, Ureidomethylmethacrylat, Amino(meth)acrylate wie 2-Aminoethyl(meth)acrylat.

Vinylester von Carbonsäuren mit 1 bis 20 C-Atomen sind z. B. Vinyllaurat, Vinylstearat, Vinylpropionat, Versaticsäurevinylester und Vinylacetat.

Als Vinylether von 1 bis 10 C-Atome enthaltende Alkohole sind z. B. Methylvinylether, Ethylvinylether, Butylvinylether, 4-Hydroxybutylvinylether, Vinylisobutylether oder Dodecylvinylether zu nennen.

Als vinylaromatische und -heteroaromatische Verbindungen kommen beispielsweise Vinyltoluol, α- und p-Methylstyrol, α-Butylstyrol, 4-n-Butylstyrol, 4-n-Decylstyrol, Styrol, Divinylbenzol, 2-Vinylpryridin, N-Vinylimidazol, N-Vinylpiperidon, N-Vinyl-2-methylimidazol und N-Vinyl-4-methylimidazol in Betracht.

Vinyllactame mit 3 bis 10 C-Atomen im Ring sind beispielsweise N-Vinylcaprolactam, N-Vinylpyrrolidon, Laurolactam, oxygenierte Purine wie Xanthin oder dessen Derivate wie 3-Methylxanthin, Hypoxanthin, Guanin, Theophyllin, Coffein, Adenin oder Theobromin.

Weiterhin können offenkettige N-Vinylamidverbindungen und N-Vinylaminverbindungen wie beispielsweise N-Vinylformamid, N-Vinyl-N-methylformamid, N-Vinylacetamid, N-Vinyl-N-methylacetamid, N-Vinyl-N-ethylacetamid, N-Vinylpropionamid, N-Vinyl-N-methylpropionamid und N-Vinylbutyramid sowie N-Vinyl-N-dimethylamin, N-Vinyl-N-methylethylamin, N-Vinyl-N-diethylamin nach dem erfindungsgemäßen Verfahren stabilisiert werden.

Die Vinylhalogenide sind mit Chlor, Fluor oder Brom substituierte ethylenisch ungesättigte Verbindungen wie beispielsweise Vinylchlorid, Vinylfluorid und Vinylidenchlorid.

Aliphatische gegebenfalls halogenierte Kohlenwasserstoffe mit 2 bis 8 C-Atomen und 1 oder 2 olefinischen Doppelbindungen sind beispielsweise Ethylen, Propen, 1-Buten, 2-Buten, Isobuten, Butadien, Isopren und Chloropren genannt.

Als Vinylidene sei beispielsweise Vinylidencyanid genannt.

Weitere polymerisationsfähige Verbindungen sind N-Vinylcaprolactam, Vinylphosphorsäuren, Vinylessigsäure, Allylessigsäure, N-Vinylcarbazol, Hydroxymethylvinylketon, N,N-Divinylethylenharnstoff, Vinylencarbonat, Tetrafluorethylen, Hexafluorpropen, Nitroethylen, Allylessigsäure, α-Chloracrylester, α-Cyanoacrylester, Methylenmalonester, α-Cyansorbinsäureester, Cyclopentadien, Cyclopenten, Cyclohexen und Cyclododecen.

In einer bevorzugten Ausführungsform des erfindungsgemäßen Verfahrens werden die Radikalfänger zur Stabilisierung von mono-, di- oder triethylenisch ungesättigten C₃-C₈-Carbonsäuren, sowie deren C₁-C₂₀-Alkylester oder N-Vinylcaprolactam, N-Vinylformamid, N-Vinylimidazol, N-Vinylpyrrolidon, Vinylphosphorsäuren, N-Vinylcarbazol, N,N-Divinylethylenharnstoff, Trimethylolpropanacrylat, Ureidomethylmethacrylat, Styrol, Butadien oder Isopren eingesetzt.

Bevorzugte ungesättigte C₃-C₈-Carbonsäuren sind beispielsweise Acrylsäure und Methacrylsäure sowie deren C₁-C₈-Alkylester wie z. B. Methyl(meth)acrylat, E-thyl(meth)acrylat, n-Butyl(meth)acrylat, 2-Ethylhexyl(meth)acrylat.

In einer bevorzugten Ausführungsform werden die erfindungsgemäßen Radikalfänger in einem Verfahren verwendet, wie es in DE-A 100 64 642 beschrieben ist. Dazu wird in einem Verfahren zur Aufarbeitung von (Meth)acrylsäure in Gegenwart mindestens eines Stabilisators ein aus der Aufarbeitung stammendes, im wesentlichen von (Meth)acrylsäure befreites, Stabilisator enthaltendes Stoffgemisch in einen Destillationsapparat geführt und ein aus diesem erhaltener, Stabilisator enthaltender Leichtsiederstrom in die Aufarbeitung rückgeführt.

Für ein solches Verfahren sind solche erfindungsgemäßen Radikalfänger besonders geeignet, deren Dampfdrücke bei 141 °C (Siedepunkt von Acrylsäure) bei Normaldruck mindestens 15 hPa betragen, bevorzugt zwischen 20 und 800 hPa, besonders bevorzugt zwischen 25 und 500 hPa, ganz besonders bevorzugt zwischen 25 und 250 hPa und insbesondere zwischen 25 und 160 hPa, sowie deren Gemische.

In einer weiteren bevorzugten Ausführungsform werden die erfindungsgemäßen Radikalfänger in einem Verfahren zur Aufarbeitung von N-Vinylmonomeren wie den genannten Vinylestern, Vinylethern, Vinylaromaten und -heteroaromaten sowie den offenkettigen V-Vinylamidverbindungen und N-Vinylaminverbindungen eingesetzt.

Selbstverständlich bezieht sich das Einsatzgebiet des erfindungsgemäßen Verfahrens auch auf Lagerung und Transport dieser polymerisationsfähigen Verbindungen.

In dieser Schrift verwendete ppm- und Prozentangaben beziehen sich, falls nicht anders angegeben, auf Gewichtsprozente und -ppm.

Die folgenden Beispiele sollen die Erfindung erläutern, aber nicht auf diese Beispiele einschränken.

### Beispiel 1

0,5 ml frisch aufgetaute und zur Entfernung des Lagerstabilisators zweifach destillierte Acrylsäure wurden unter Luftatmosphäre in 1,8 ml Ampullen abgefüllt.

Die Proben wurden alle im Umlufttrockenschrank bei 120 °C gelagert.

In jeder Testreihe wurden von jeder Acrylsäureprobe jeweils 3 Ampullen befüllt und getestet, der Mittelwert der Zeit bis zur vollständigen Polymerisation wurde visuell erfasst.

Die durchschnittliche Standardabweichung innerhalb einer Versuchsreihe lag bei ca. 2-4%.

Die Konzentrationen betrugen, soweit nicht anders angegeben, 25 ppm Radikalfänger plus 10 ppm Phenothiazin (PTZ).

Die relative Wirksamkeit berechnet sich aus dem Quotienten aus der Zeit bis zur Polymerisation der Probe aus Radikalfänger und PTZ und der Zeit bis zur Polymerisation der Referenzprobe. Als Referenzprobe wurde reines PTZ verwendet, die relative Wirksamkeit der Referenzprobe beträgt demnach 1,0.

Die Ergebnisse sind in Tabelle 3 zusammengefasst.

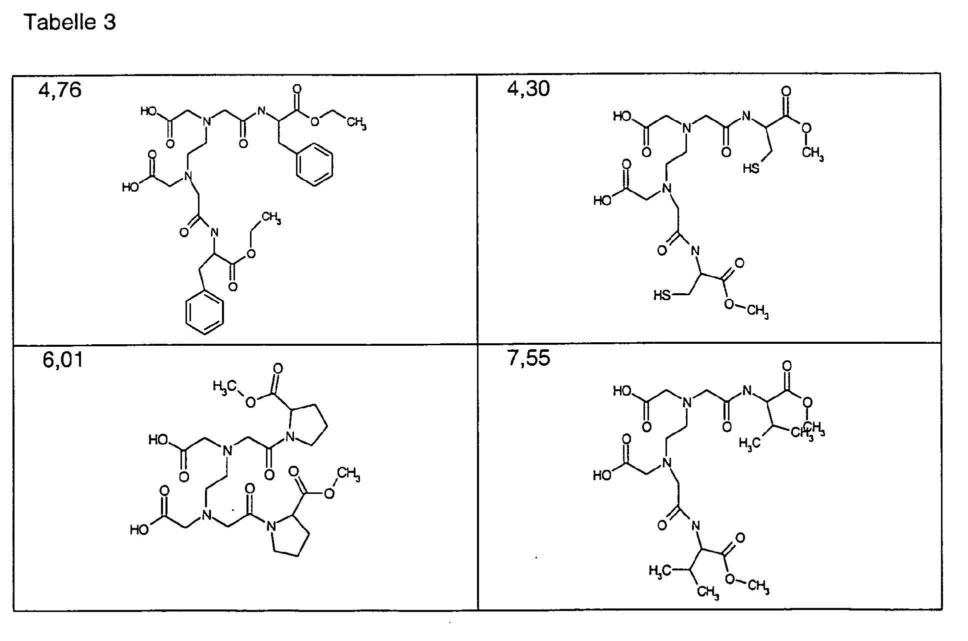

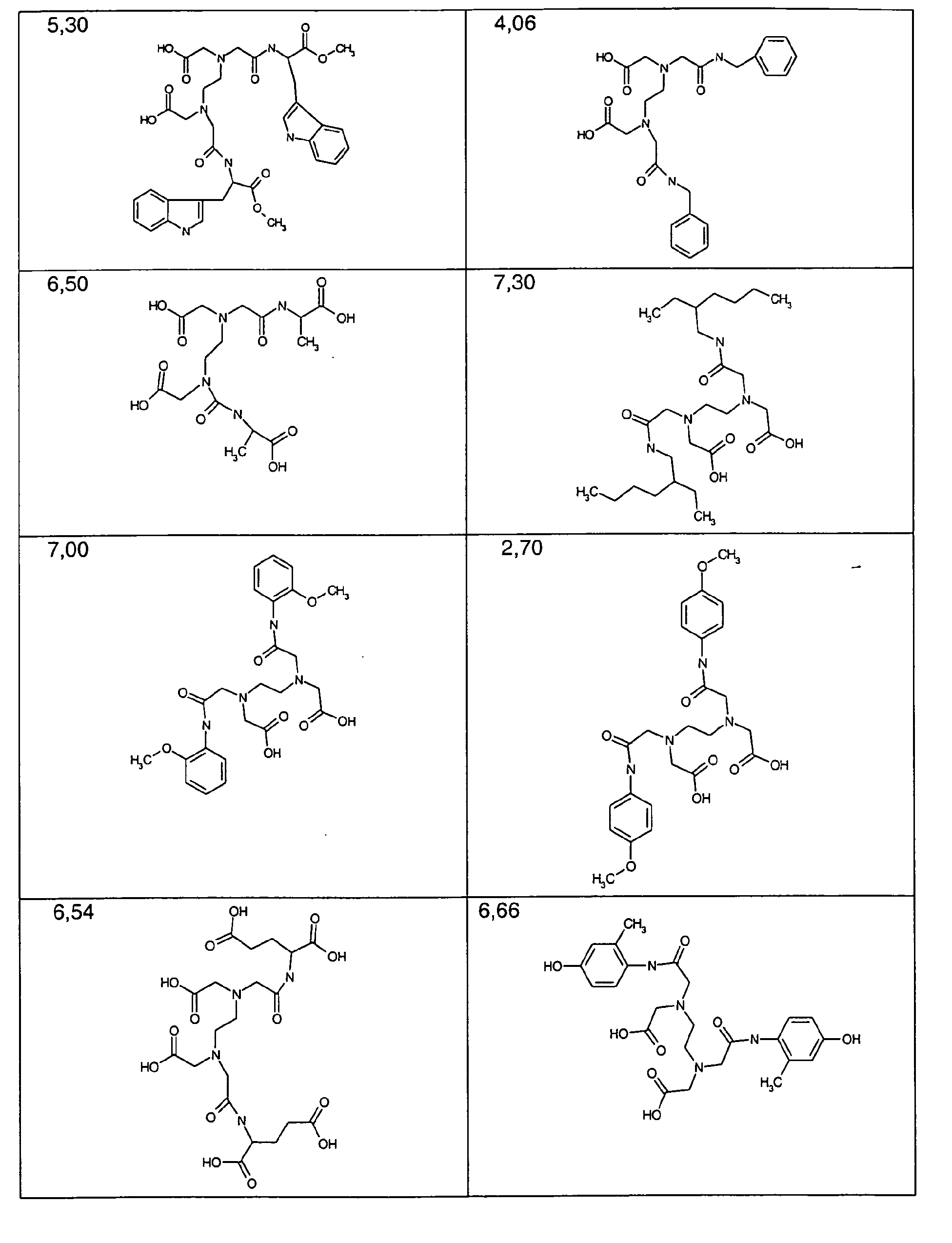

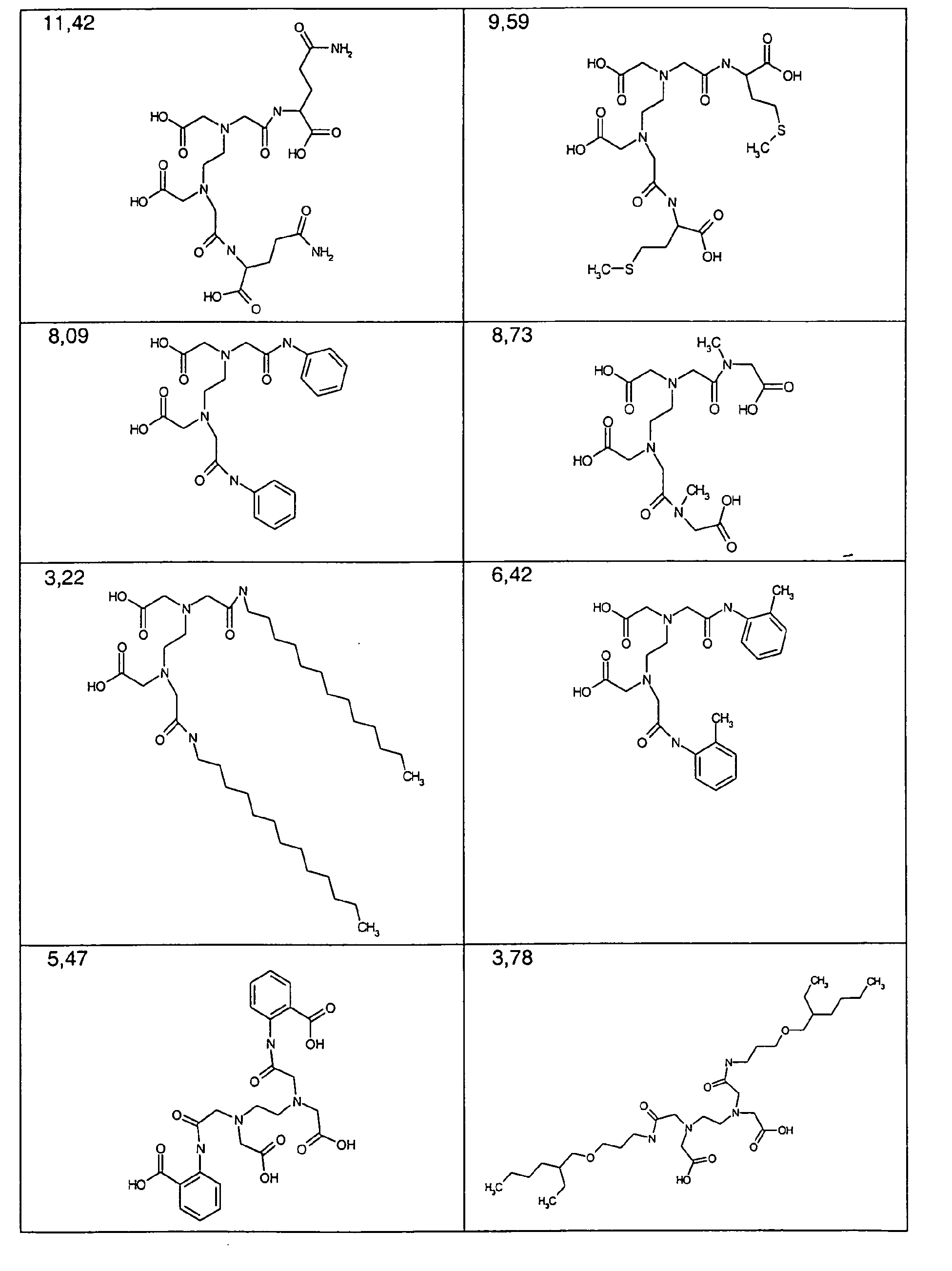

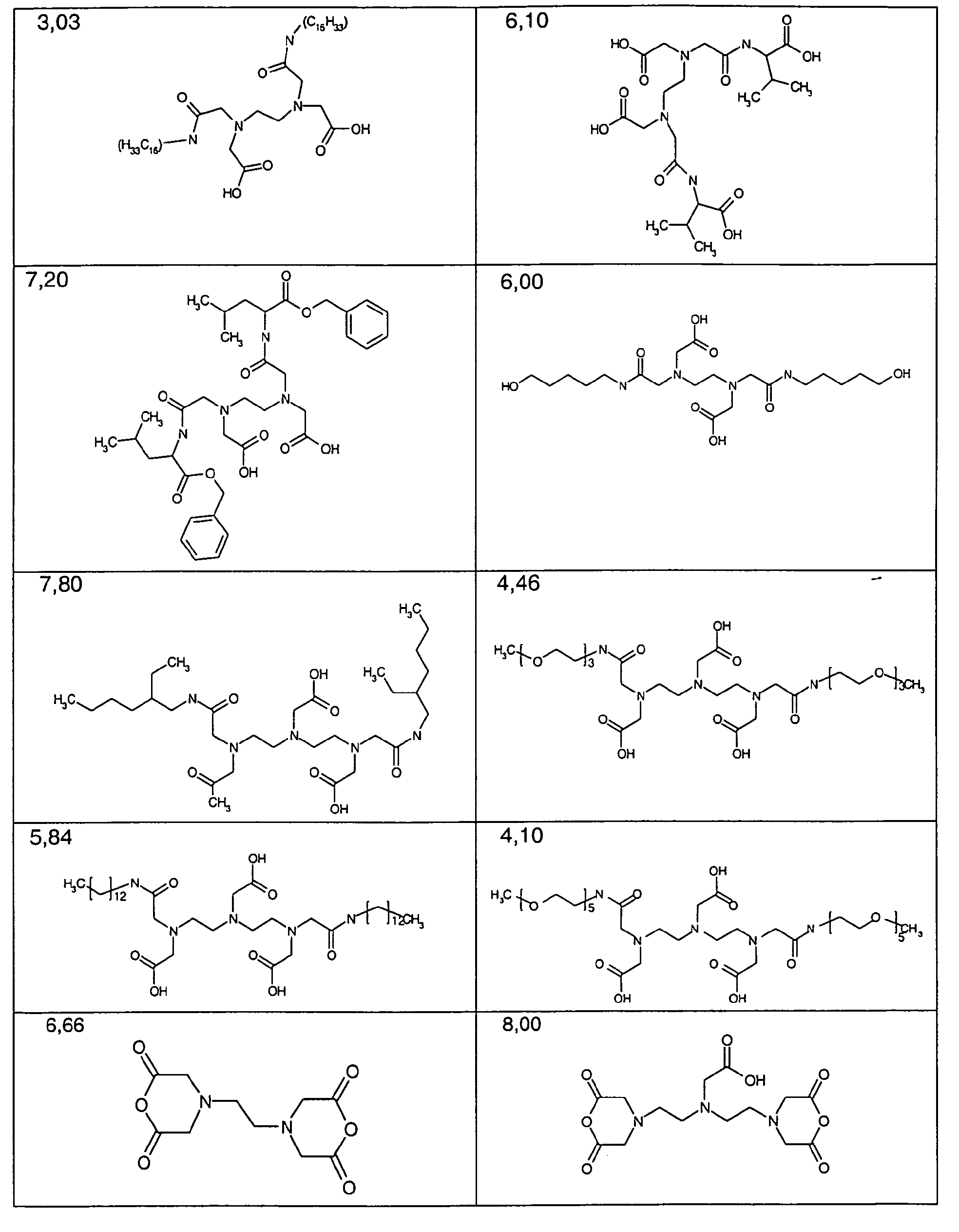

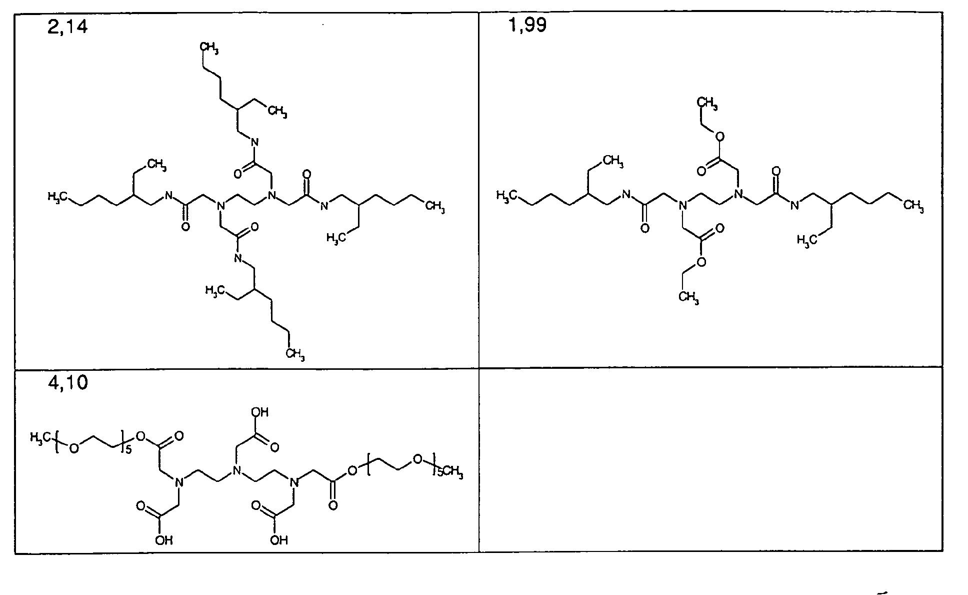

### Beispiel 2

In einem 250 ml Rundkolben wurden 90 ml des Monomers mit der gewünschten Menge eines Stabilisators eingefüllt und mit der entsprechenden Atmosphäre gespült. Der Kolben wurde auf die gewünschte Lagertemperatur erhitzt. Es wurden in regelmäßigen Abständen Proben entnommen. Diese wurden ramanspektroskopisch auf den Polymergehalt untersucht. Der Kurvenverlauf relativ zur unstabilisierten Probe zeigt die Wirksamkeit des eingesetzten Stabilisatorsystems.

Als Referenz wurde ein Gemisch von N-Vinylpyrrolidon (NVP) und Pyrrolidon (1:1) eingesetzt und mit 25 ppm PTZ versetzt. Die Probe wurde bei 150 °C unter einer Luftatmosphäre untersucht. Es erfolgte schnelle Polymerbildung (PVP = Polyvinylpyrrolidon). Die Ergebnisse sind in Tabelle 4 dargestellt.

| Zeit [min] | PVP [Gew-%] 25 ppm PTZ |
|---|---|
| 0 | 0,0 |
| 15 | 4,4 |
| 30 | 9,4 |
| 45 | 13,1 |
| 60 | 16,6 |
| 75 | 19,0 |
| 90 | 21,8 |
| 105 | 23,6 |
| 120 | 25,0 |
| 135 | 27,4 |
| 150 | 29,2 |
| 165 | 30,8 |
| 180 | 32,7 |

Eine Mischung von jeweils 25 ppm Radikalfänger (Tabelle 3 aus Beispiel 1) und 25 ppm PTZ wurde zu einem Gemisch aus N-Vinylpyrrolidon und Pyrrolidon (1:1) gegeben und bei identischen Bedingungen erhitzt. Dabei konnten keine signifikanten Unterschiede zwischen den in Beispiel 1 verwendeten Radikalfängern ermittelt werden. In Tabelle 5 sind die Ergebnisse eines Radikalfängers (Tabelle 3 aus Beispiel 1, 1. Eintrag) zusammengefasst. Die Bildung von PVP in Gegenwart eines erfindungsgemäßen Radikalfängers und PTZ konnte erfolgreich unterdrückt werden.

| Zeit [min] | PVP [Gew-%] 25 ppm PTZ 25 ppm Rf ¹⁾ |
|---|---|
| 0 | 0,0 |
| 15 | 0,3 |
| 30 | 0,1 |
| 45 | 0,2 |
| 60 | 0,7 |
| 75 | 0,3 |
| 90 | 0,5 |
| 105 | 0,7 |
| 120 | 1,1 |
| 135 | 2,0 |
| 150 | 1,5 |
| 165 | 2,3 |
| 180 | 2,7 |

| | |
|---|---|
| ¹⁾ Rf = Radikalfänger | |

Anschließend wurde anhand dieses Beispiels der Einfluss verschiedener Konzentrationen an PTZ und erfindungsgemäßem Radikalfänger untersucht. Die Ergebnisse sind in Tabelle 6 veranschaulicht. Daraus wird deutlich, dass ab einer Menge von jeweils 25 ppm PTZ und Radikalfänger kein signifikanter Unterschied in der Polymerisationsinhibierung erkennbar ist.

| Zeit [min] | PVP [Gew-%] unstabilisiert | PVP [Gew-%] 12,5 ppm PTZ 12,5 ppm Rf ¹⁾ | PVP [Gew-%] 25 ppm PTZ 25 ppm Rf ¹⁾ | PVP [Gew-%] 50 ppm PTZ 50 ppm Rf ¹⁾ | PVP [Gew-%] 100 ppm PTZ 100 ppm Rf ¹⁾ |
|---|---|---|---|---|---|
| 0 | 0,6 | 0,4 | 0,0 | 0,1 | 0,3 |
| 15 | 35,2 | 1,1 | 0,3 | 0,0 | 0,0 |
| 30 | 42,1 | 1,9 | 0,1 | 0,3 | 0,2 |
| 45 | 46,2 | 2,9 | 0,2 | 0,1 | 0,2 |
| 60 | 48,1 | 5,2 | 0,7 | 0,4 | 0,6 |
| 75 | 49,7 | 5,6 | 0,3 | 0,5 | 0,7 |
| 90 | 50,1 | 6,5 | 0,5 | 1,0 | 0,6 |
| 105 | 51,2 | 7,7 | 0,7 | 0,8 | 0,9 |
| 120 | 51,8 | 7,9 | 1,1 | 1,2 | 1,0 |
| 135 | 52,6 | 8,7 | 2,0 | 1,4 | 1,5 |
| 150 | 53,2 | 9,0 | 1,5 | 1,1 | 1,9 |
| 165 | 53,3 | 9,3 | 2,3 | 1,3 | 2,1 |
| 180 | 54,1 | 9,7 | 2,7 | 1,8 | 2,7 |

| | | | | | |
|---|---|---|---|---|---|
| ¹⁾ Rf = Radikalfänger | | | | | |

## Patentansprüche

1. Verfahren zur Stabilisierung polymerisationsfähiger Verbindungen gegen Polymerisation bei der Aufarbeitung, Lagerung und/oder Transport, **dadurch gekennzeichnet, dass** man wenigstens einen Radikalfänger, der mindestens zwei Glycineinheiten enthält, verwendet, mit der Maßgabe, dass der Radikalfänger nicht ausgewählt ist aus Ethylendiamintetraessigsäure (EDTA), Diethylentriaminpentaessigsäure (DTPA), trans-1,2-Cyclohexandiamintetraessigsäure (CYDTA) und deren Alkali- und Erdalkalisalze.

2. Verfahren nach Anspruch 1, **dadurch gekennzeichnet, dass** man wenigstens einen Radikalfänger, der mindestens zwei Glycineinheiten enthält, verwendet, mit der Maßgabe, dass der Radikalfänger nicht ≥ 2 der folgenden Struktureinheiten aufweist: 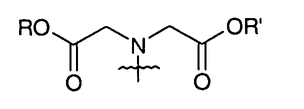 worin
R, R' unabhängig voneinander Wasserstoff oder Metall
sein können.

3. Verfahren nach Anspruch 1 oder 2 , **dadurch gekennzeichnet, dass** man wenigstens einen Radikalfänger, der mindestens 2 Glycineinheiten und mindestens eine Amid- und/oder Estereinheit enthält, verwendet.

4. Verfahren nach den Ansprüchen 1 bis 3, **dadurch gekennzeichnet, dass** man wenigstens einen Radikalfänger der Formel (I) verwendet 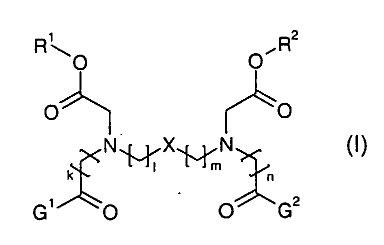 worin,
G¹ NR³R⁴ oder OR⁷ sein kann,
G² NR⁵R⁶ oder OR⁸ sein kann,
R¹ und R² unabhängig voneinander Wasserstoff, C₁-C₂₀-Alkyl, C₁-C₂₀-Alkylcarbonyl, C₂-C₂₀-Alkenyl, C₂-C₂₀-Alkenylcarbonyl, C₂-C₂₀-Alkinyl, C₂-C₂₀-Alkinylcarbonyl, C₃-C₁₅-Cycloalkyl, C₅-C₁₅-Cycloalkylcarbonyl, Aryl, Arylcarbonyl oder Heterocyclen sein können,
R³ und R⁵ unabhängig voneinander Wasserstoff, C₁-C₂₀-Alkyl, C₁-C₂₀-Alkylcarbonyl, C₂-C₂₀-Alkenyl, C₂-C₂₀-Alkenylcarbonyl, C₂-C₂₀-Alkinyl, C₂-C₂₀-Alkinylcarbonyl, C₃-C₁₅-Cycloalkyl, C₅-C₁₅-Cycloalkylcarbonyl, Aryl, Arylcarbonyl oder Heterocyclen sein können,
R⁴ und R⁶ unabhängig voneinander C₁-C₂₀-Alkyl, C₁-C₂₀-Alkylcarbonyl, C₂-C₂₀-Alkenyl, C₂-C₂₀-Alkenylcarbonyl, C₂-C₂₀-Alkinyl, C₂-C₂₀-Alkinylcarbonyl, C₃-C₁₅-Cycloalkyl, C₅-C₁₅-Cycloalkylcarbonyl, Aryl, Arylcarbonyl oder Heterocyclen sein können,
R⁷ und R⁸ unabhängig voneinander C₁-C₂₀-Alkyl, C₁-C₂₀-Alkylcarbonyl, C₂-C₂₀-Alkenyl, C₂-C₂₀-Alkenylcarbonyl, C₂-C₂₀-Alkinyl, C₂-C₂₀-Alkinylcarbonyl, C₃-C₁₅-Cycloalkyl, C₅-C₁₅-Cycloalkylcarbonyl, Aryl, Arylcarbonyl oder Heterocyclen sein können,
X C₁-C₂₀-Alkyl, NCH₂COOR⁹, NR¹⁰, O, S, PR¹¹, Se, SiOR¹²R¹³ oder Aryl sein kann, worin R⁹ bis R¹³ unabhängig voneinander Wasserstoff oder C₁-C₂₀-Alkyl sein können, und
k, l, m, n unabhängig voneinander für Zahlen von 0 bis 20 stehen können.

5. Verfahren nach den Ansprüchen 1 bis 4, **dadurch gekennzeichnet, dass** R¹ und R² gleich und Wasserstoff oder C₁-C₂₀-Alkyl sind.

6. Verfahren nach den Ansprüchen 1 bis 5, **dadurch gekennzeichnet, dass** R³ und R⁵ gleich und Wasserstoff, C₁-C₂₀-Alkyl oder C₁-C₂₀-Alkylcarbonyl sind.

7. Verfahren nach den Ansprüchen 1 bis 6, **dadurch gekennzeichnet, dass** R⁴ und R⁶ gleich und C₁-C₂₀-Alkyl, C₁-C₂₀-Alkylcarbonyl, Aryl, C₂-C₂₀-Alkenyl, C₂-C₂₀-Alkenylcarbonyl, C₂-C₂₀-Alkinyl oder C₂-C₂₀-Alkyinylcarbonyl sind.

8. Verfahren nach den Ansprüchen 6 oder 7, **dadurch gekennzeichnet, dass** R³ und R⁵ Wasserstoff und R⁴ und R⁶ ausgewählt sind aus Phenyl, Benzyl, p-Methoxyphenyl, o-, m- oder p- Hydroxyphenyl, 1-Hydroxyhexyl, Methyl, Ethyl, Propyl, Butyl, Ethylenglykol, Diethylenglykol, Triethylenglykol, Ethoxylat mit 4 bis 10 EO-Einheiten, Ethylendiamin, Diethylentriamin, Triethylentetraamin und Aminosäuren.

9. Verfahren nach den Ansprüchen 1 bis 5, **dadurch gekennzeichnet, dass** R⁷ und R⁸ gleich und C₁-C₂₀-Alkyl, C₁-C₂₀-Alkylcarbonyl, Aryl, C₂-C₂₀-Alkenyl, C₂-C₂₀-Alkenylcarbonyl, C₂-C₂₀-Alkinyl oder C₂-C₂₀-Alkyinylcarbonyl sind.

10. Verfahren nach Anspruch 9, **dadurch gekennzeichnet, dass** R⁷ und R⁸ ausgewählt sind aus Phenyl, Benzyl, p-Methoxyphenyl, o-, m- oder p- Hydroxyphenyl, 1-Hydroxyhexyl, Methyl, Ethyl, Propyl, Butyl, Ethylenglykol, Diethylenglykol, Triethylenglykol, Ethoxylat mit 4 bis 10 EO-Einheiten, Ethylendiamin, Diethylentriamin, Triethylentetraamin und Aminosäuren.

11. Verfahren nach den Ansprüchen 1 bis 10, **dadurch gekennzeichnet, dass** X C₁-C₂₀-Alkyl oder CH₂NCOOR⁹ ist.

12. Verfahren nach den Ansprüchen 1 bis 11, **dadurch gekennzeichnet, dass** man wenigstens eine der folgenden Verbindungen einsetzt:

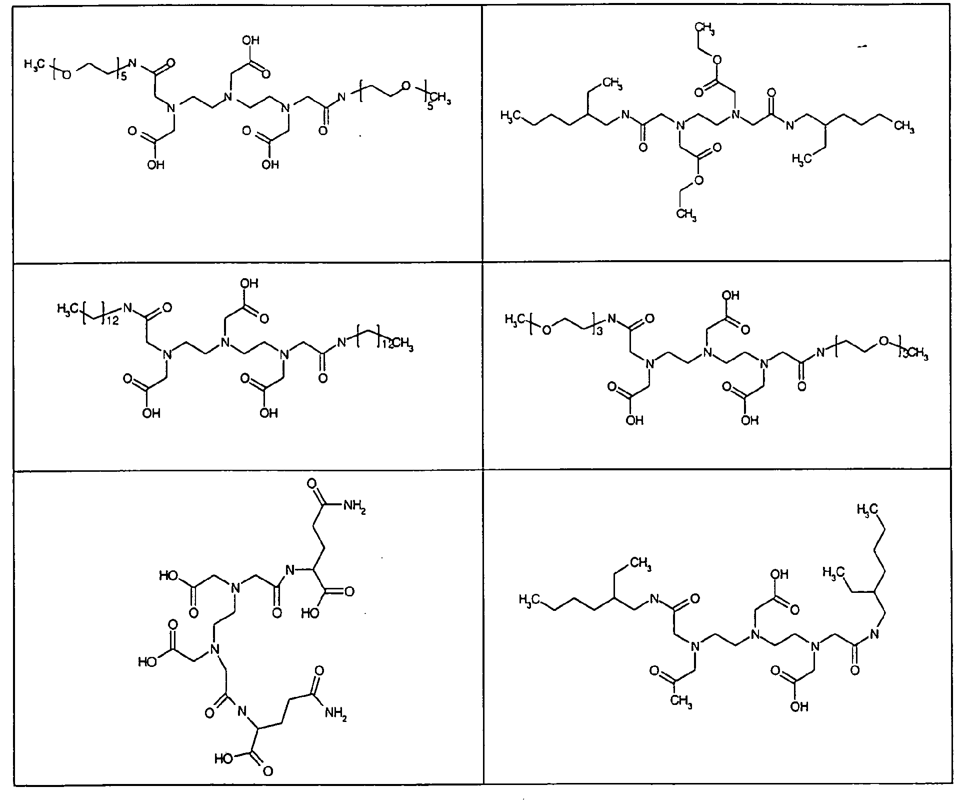

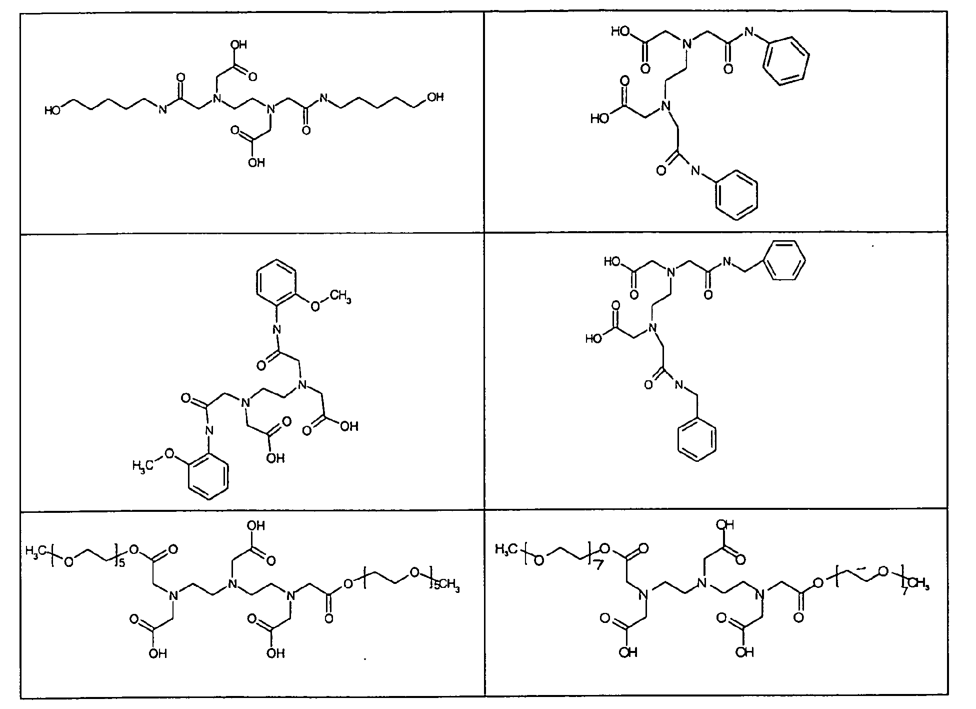

13. Verfahren nach den Ansprüchen 1 bis 12, **dadurch gekennzeichnet, dass** man 0,1 bis 1000 ppm des Radikalfängers oder eines Radikalfängergemisches bezogen auf die polymerisationsfähige Verbindung einsetzt.

14. Verfahren nach den Ansprüchen 1 bis 13, **dadurch gekennzeichnet, dass** man wenigstens einen Costabilisator einsetzt.

15. Verfahren nach Anspruch 14, **dadurch gekennzeichnet, dass** der Costabilisator ausgewählt ist aus der Gruppe der sauerstoffhaltigen Gase, phenolischen Verbindungen, Chinone und Hydrochinone, N-Oxylverbindungen, aromatischen Amine, Phenylendiamine, Imine, Sulfonamide, Oxime, Hydroxylamine, Harnstoffderivate, phosphorhaltigen Verbindungen, schwefelhaltigen Verbindungen, Komplexbildner auf Basis von Tetraazannulen und Metallsalze, sowie gegebenenfalls Mischungen davon.

16. Verfahren nach Anspruch 14 oder 15, **dadurch gekennzeichnet, dass** man als Costabilisator Phenothiazin, Hydrochinon, Hydrochinonmonomethylether, 2,2,6,6-Tetramethyl-piperidin-N-oxyl, 4-Hydroxy-2,2,6,6-tetramethyl-piperidin-N-oxyl, 4-Oxo-2,2,6,6-tetramethyl-piperidin-N-oxyl, N,N'-Di-sec.-butyl-pphenylendiamin, Cer(III)acetat, Cer(III)ethylhexanoat, sauerstoffhaltige Gase und/oder Mischungen davon einsetzt.

17. Verfahren nach einem der vorstehenden Ansprüche, **dadurch gekennzeichnet, dass** die polymerisationsfähige Verbindung mindestens eine ethylenisch ungesättigte Gruppe enthält.

18. Verfahren nach Anspruch 17 **dadurch gekennzeichnet, dass** die poylmerisationsfähige Verbindung ausgewählt ist aus der Gruppe der mono-, di- oder triethylenisch ungesättigten C₃-C₈-Carbonsäuren, C₁-C₂₀-Ester, -Amide, -Nitrile und - Anhydride dieser mono-, di- oder triethylenisch ungesättigten C₃-C₈-Carbonsäuren, Vinylester von bis zu 20 C-Atome enthaltenden Carbonsäuren, Vinylether von 1 bis 10 C-Atome enthaltenden Alkoholen, Vinylaromaten und - heteroaromaten mit bis zu 20 C-Atomen, Vinyllactame mit 3 bis 10 C-Atomen im Ring, offenkettigen N-Vinylamidverbindungen und N-Vinylaminverbindungen, Vinylhalogenide, aliphatischen gegebenenfalls halogenierten Kohlenwasserstoffe mit 2 bis 8 C-Atomen und 1 oder 2 Doppelbindungen, Vinylidene oder Mischungen dieser Monomeren.

19. Verfahren nach Anspruch 17 oder 18, **dadurch gekennzeichnet, dass** man als polymerisationsfähige Verbindung mono-, di- oder triethylenisch ungesättigte C₃-C₈-Carbonsäuren, C₁-C₂₀-Ester dieser mono-, di- oder triethylenisch ungesättigten C₃-C₈-Carbonsäuren, Vinylester von bis zu 20 C-Atome enthaltenden Carbonsäuren, Vinylether von 1 bis 10 C-Atome enthaltenden Alkoholen, Vinylaromaten und -heteroaromaten mit bis zu 20 C-Atomen, Vinyllactame mit 3 bis 10 C-Atomen im Ring, offenkettigen N-Vinylamidverbindungen oder N-Vinylaminverbindungen einsetzt.

20. Verfahren nach den Ansprüchen 17 bis 19 **dadurch gekennzeichnet, dass** man als polymerisationsfähige Verbindung (Meth)acrylsäure, (Meth)acrylsäureester, N-Vinylcaprolactam, N-Vinylformamid, N-Vinylimidazol, N-Vinylpyrrolidon, Vinylphosphorsäuren, N-Vinylcarbazol, N,N-Divinylethylenharnstoff, Trimethylolpropantriacrylat, Ureidomethylmethacrylat, Styrol, Butadien oder Isopren einsetzt.

21. Stabilisatormischung enthaltend
i) wenigstens einen Radikalfänger, der mindestens zwei Glycineinheiten enthält, mit der Maßgabe, dass der Radikalfänger nicht ausgewählt ist aus Ethylendiamintetraessigsäure (EDTA), Diethylentriaminpentaessigsäure (DTPA), trans-1,2-Cyclohexandiamintetraessigsäure (CYDTA) und deren Alkali- und Erdalkalisalze und
ii) wenigstens einen weiteren Stabilisator oder Costabilisator.

22. Stoffgemisch enthaltend eine Stabilisatormischung nach Anspruch 21 und wenigstens eine polymerisationsfähige Verbindung.

23. Verwendung einer Stabilisatormischung nach Anspruch 21 zur Stabilisierung polymerisationsfähiger Verbindungen gegen Polymerisation bei der Aufarbeitung, Lagerung und/oder Transport.
